# EUROPEAN PATENT SPECIFICATION

(11) **EP 4 453 552 B1**
(45) Date of publication and mention of the grant of the patent: **22.10.2025**
(21) Application number: 22840742.5
(22) Date of filing: 21.12.2022
(51) Int. Cl.: G01N 27/414

(54) **METHOD FOR DETERMINING THE CONCENTRATION OF AN ANALYTE IN A SAMPLE**
VERFAHREN ZUR BESTIMMUNG DER KONZENTRATION EINES ANALYTEN IN EINER PROBE
PROCÉDÉ POUR DÉTERMINER LA CONCENTRATION D'UN ANALYTE DANS UN ÉCHANTILLON

(30) Priority: 22.12.2021 EP 21216913
(43) Date of publication of application: 30.10.2024
(73) Proprietor: Roche Diagnostics GmbH, 68305 Mannheim (DE); F. Hoffmann-La Roche AG, 4070 Basel (CH)
(72) Inventor: BRADBURY, Christopher, 6343 Rotkreuz ZG (CH); GUTIÉRREZ-SANZ, Oscar, 68305 Mannheim (DE)
(74) Representative: Altmann Stößel Dick Patentanwälte PartG mbB
(86) International application number: PCT/EP2022/087181
(87) International publication number: WO 2023/118264

(56) References cited:
- JP-A- 2013 092 479
- US-A1- 2019 339 229
- MASSEY R ET AL: "Label-free Detection of Dopamine Using Aptamer Enhanced Organic-Electrolyte Gated FET Sensor", 2019 IEEE INTERNATIONAL CONFERENCE ON FLEXIBLE AND PRINTABLE SENSORS AND SYSTEMS (FLEPS), IEEE, 8 July 2019 (2019-07-08), pages 1 - 3, XP033592821, [retrieved on 20190807], DOI: 10.1109/FLEPS.2019.8792324
- MELZER K ET AL: "Enzyme assays using sensor arrays based on ion-selective carbon nanotube field-effect transistors", BIOSENSORS AND BIOELECTRONICS, ELSEVIER SCIENCE LTD, UK, AMSTERDAM , NL, vol. 84, 23 April 2016 (2016-04-23), pages 7 - 14, XP029536910, ISSN: 0956-5663, DOI: 10.1016/J.BIOS.2016.04.077
- TATAVARTHI SAI SUDHEER ET AL: "Rapid and Highly Sensitive Extended Gate FET-Based Sensors for Arsenite Detection Using a Handheld Device Rapid and Highly Sensitive Extended Gate FET-Based Sensors for Arsenite Detection Using a Handheld Device", ECS J. SOLID STATE SCI. TECHNOL, 12 August 2020 (2020-08-12), pages 115014, XP055928124, Retrieved from the Internet <URL:https://iopscience.iop.org/article/10.1149/2162-8777/abab18/pdf> [retrieved on 20220605]

## Description

### Technical Field

The invention relates to a method of determining the concentration of at least one analyte in a sample. The description further relates to a sensor device for determining the concentration of at least one analyte in a sample. As an example, the devices and methods of the present invention may be used for diagnostic purposes such as in clinical or laboratory analytics or for home monitoring purposes. The methods and devices of the present invention may specifically be used for determining the concentration of an analyte, such as for instance potassium, in a bodily fluid or in another liquid. However, other applications are also feasible.

### Background art

A variety of sensor devices for sensing analytes have been described so far. Such sensor devices in many cases rely on field effect transistor based measurements for qualitatively identifying the analyte or for quantitatively determining the concentration of the analyte. The presence of an analyte results in a change in the electric field and consequently in a change in the charge carrier density within the conducting channel of the field effect transistor. This again induces a measurable change in a current or a potential between source and drain of the field effect transistor. Often, the source and drain contacts are protected from contact with the analyte and the gate surface is functionalized for a specific binding of the analyte. However, other options are feasible as well.

EP 1 348 951 A1 discloses a sensing device comprising a sensing layer having at least one functional group that binds to the semiconducting channel layer and at least another functional group that serves as a sensor, a semiconducting channel layer having a first surface and a second surface, which is opposite to said surface, a drain electrode, a source electrode, a gate electrode, characterized in that said source electrode, said drain electrode and said gate electrode are placed on the first surface of said semi-conducting channel layer and that said sensing layer is on the surface of said semiconducting channel layer, said sensing layer being in contact with the semi-conducting channel layer and said semi-conducting channel layer having a thickness below 5000nm.

JP S61153559 A discloses a semiconductor enzyme sensor with the purpose to permit the easy reduction of the size of a semiconductor enzyme sensor and the formation of multisensors and to extend the life thereof by using a water absolute photosensitive resin of polyvinyl pyrrolidone-diazide to form directly an enzyme immobilized film to the ion sensitive surface of a pH-ISFET. The pH-ISFET element as a substratum electrode of the enzyme sensor is provided with sources and drains, a pseudo reference electrode and lead wires, and is formed as a composite type element. The respective elements sense respectively independently hydrogen ions to permit the measurement of the pH in a solution. The film immobilized with the enzyme of which the pH changes by the reaction with a substrate is mounted to the channel part of one element, and is not mounted to the other element. The water soluble photosensitive resin of polyvinyl pyrrolidone-diazide is coated to cover the ion sensitive surface of the channel part of the element consisting of the source and the drain.

JP S6029658 A discloses a urea sensor with the purpose to permit easy miniaturization and manufacture of multisensors with a simple process and to extend the life of the sensors by forming directly an urease-immobilized film on a pH-ISFET by using a photosensitive resin. A pH-ISFET element which is an underlying electrode is pro-vided with sources and drains, a pseudo-reference electrode and lead wires 7. This pH-ISFET element is the composite type pH-ISFET element constituted of one pH-ISFET(A) consisting of the source and drain and another pH-ISFET(B) consisting of the source and the drain as well as the (pseudo) reference electrode . The glucose sensor is manufactured by a method for mounting an ureaseimmobilized film on the part of the pH-ISFET(A) and not mounting sand film on the another pH-ISFET(B). If there is urea in the sample solution, the urea is decomposed and the pH of the urease-immobilized film generates a difference in the pH of the sample solution itself monitored by the pH- ISFET(B) having no urease-immobilized film.

US 2016/047775 A1 discloses embodiments of sensing devices including one or more integrated circuit (IC) die, a housing, and a fluid barrier material. Each IC die includes an electrode-bearing surface and a contact surface. One of the die includes an SFET with a sensing electrode proximate to the electrode-bearing surface. The same or a different die includes a reference electrode proximate to the electrode-bearing surface. The die(s) also include IC contacts at the contact surface(s), and conductive structures coupled between the SFET, the reference electrode, and the IC contacts. The housing includes a mounting surface, and housing contacts formed at the mounting surface. The IC contacts are coupled to the housing contacts. The fluid barrier material is positioned between the mounting surface and the IC die. The fluid barrier material provides a fluid barrier between the IC and housing contacts and a space that encompasses the sensing electrode and the reference electrode.

CN 105301079 A discloses a semiconductor device for detecting the ionic activity of an object to be detected and a detection method thereof. The semiconductor device comprises a substrate, a source and a drain, the source and the drain are arranged on the substrate, the semiconductor device also comprises a first ionsensitive membrane and a second ion-sensitive membrane which have different sensitivities to the ionic activity of the object to be detected, moreover, the object to be detected is arranged between the first ionsensitive membrane and the second ion-sensitive membrane, the first ion-sensitive membrane is arranged on the substrate, the second ion-sensitive membrane is connected with a grid power supply, and in a preferred embodiment, a comb capacitor is also introduced. The semiconductor device for detecting the ionic activity of the object to be detected, which adopts the structure, and the detection method thereof dispense a reference electrode, and introduce the two different ion-sensitive membranes to accurately measure the ionic activity of the object to be detected, the structure is simple, the cost is low, and the semiconductor device has a wide application range.

EP 3 588 075 A1 discloses a high-sensitivity biosensor. The object of the invention is to increase the detection specificity of biosensors. The invention provides a biosensor characterized in that it comprises an identifier substance that can bind to a detection target substance and an electrode that takes the charge of said identifier substance, wherein the biosensor detects the change in the charge density of said electrode generated by the binding of said detection target substance with said identifier substance, the surface of said electrode is coated with polycatecholamine, all or a part of said electrode surface coated with polycatecholamine further has a polymer layer formed thereon which has a molecular imprint having a structure complementary to the molecular structure of the detection target substance formed therein, said polymer layer comprises said identifier substance, and said polymer layer is an ultrathin film layer.

JP 2013 092479 A discloses a biosensor measuring apparatus including a biosensor unit, a measuring unit, and a controlling unit. The biosensor unit includes: a substrate; a drain electrode and a source electrode; a semiconductor film arranged on the substrate; an insulator film arranged on the semiconductor film; a sensitive film arranged on the insulator film for placing a measurement target object thereon; a reference electrode for making contact with the measurement target object and for receiving variable reference voltage; and a barrier. The measuring unit includes: an electric current source coupled to the drain electrode to output a constant electric current; a reference potential unit coupled to the source electrode; a voltmeter for measuring output voltage while setting a coupling portion of the drain electrode and the electric current source as output terminal; and a reference voltage source for applying reference voltage to the reference electrode. The controlling unit includes: a measurement setting unit for controlling measurement by the measuring unit; and a storage unit for storing the output voltage measured by the voltmeter.

US 2016/0169835 A1 discloses a biosensor for performing analysis based on a sample noninvasively collected from a human body. The biosensor comprises an identification substance that binds to a substance to be detected, and an electrode charged with a charge of the identification substance. The biosensor further comprises an inhibitor that inhibits a substance not to be detected from attaching to at least one of the identification substance and the electrode. The biosensor detects a change in a charge density of the electrode caused by binding of the substance to be detected to the identification substance.

US 2019/0339229 A1 discloses a semiconductor device comprising a first field effect transistor (FET) connected in series to a second FET, and a third FET connected in series to the first FET and the second FET. The semiconductor device further includes bias circuitry coupled to the first FET and the second FET, and an output conductor coupled to a terminal of the second FET, wherein the output conductor obtains an output signal from the second FET that is independent of the first FET.

Tatavarthi, S. S. et al. describe in "Rapid and Highly Sensitive Extended Gate FET-Based Sensors for Arsenite Detection Using a Handheld Device", ECS Journal of Solid State Science and Technology, 2020, Vol. 9, No. 11, 115014, a FET based ion selective sensor for detecting Arsenite (As(III)) ions by a hand held device. The selective sensor shows higher sensitivity (35.19 mV/log[As3+]) than the ideal Nernstian slope. The dynamic range of the Arsenite based ion sensor is from 10⁻¹⁰ M to 10⁻⁴ M. The detection limit of this FET based ion selective sensor is below 10⁻¹⁰ M.

Schmoltner, K. et al. describe in "Electrolyte-Gated Organic Field~Effect Transistor for Selective Reversible Ion Detection", Advanced Materials, Volume 25, Issue 47, December 17, 2013, Pages 6895-6899, an ion-sensitive electrolyte-gated organic field-effect transistor for selective and reversible detection of sodium (Na+) down to 10⁻⁶ M.

Melzer, K. et al. describe in "Enzyme assays using sensor arrays based on ion-selective carbon nanotube field-effect transistors", Biosensors and Bioelectronics, Volume 84, October 15, 2016, Pages 7-14, a sensors based on spray-coated electrolyte-gated carbon nanotube field-effect transistor for a selective and direct detection of the products of an enzyme-substrate interaction, here the for metabolic processes important urea-urease system. The selective and direct detection is achieved by immobilizing the enzyme urease via certain surface functionalization techniques on the sensor surface and further modifying the active interfaces with polymeric ion-selective membranes as well as pH-sensitive layers.

Generally, different options exist for measuring a current change or a potential change between source and drain in a field effect transistor based sensor device. A first common option is to obtain a transfer curve of the field effect transistor, wherein the drain-source current I_{DS} is measured as a function of the applied gate potential V_{G} while keeping the drain-source potential V_{DS} constant. Usually, several transfer curves are recorded over time in order to monitor changes in the electrical field. The range of the potentials to be swept and the sampling rate determine the time resolution of the measurement. A second common option is to monitor the drain-source current I_{DS} over time while keeping the drain-source potential V_{DS} and gate potential V_{G} fixed, which allows a high time resolution.

Despite the advantages achieved by the known devices and methods, various technical challenges still remain. Specifically, for the first above-mentioned option data processing typically is rather complex. On the other hand, the second above-mentioned option typically has a disadvantage in multiplexing, because only one gate potential V_{G} can be applied on one sample at a time. Further, for the second above-mentioned option, the output of the measurement is a current which would need to be converted into a potential for comparing the data derived from different field effect transistors. Generally, different measurement situations may require different measurement setups for best performance. Thus, for varying measurement situations, the measurement setup may have to be changed, which can be a laborious process, specifically when changing hardware components.

### Problem to be solved

It is therefore desirable to provide a method of determining the concentration of at least one analyte in a sample that, at least partially, addresses the above-mentioned challenges of known devices and methods of similar kind. Specifically, it is desirable to provide a widely applicable method for determining the named concentration with high performance and reproducibility, wherein the high performance and reproducibility can at best be achieved by resource-saving measures that are quickly adaptable to varying measurement situations. More specifically, it is desirable to provide an approach allowing for high time resolution measurements and efficient multiplexing of different sensor devices.

### Summary

This problem is addressed by a method of determining the concentration of at least one analyte in a sample as defined in independent claim 1. Advantageous embodiments that might be realized are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The term "analyte" as used herein is a broad term and is to be given its ordinary and customary meaning to a person of ordinary skill in the art and is not to be limited to a special or customized meaning. The term specifically may refer, without limitation, to an arbitrary chemical or biological substance or species, such as an ion, an atom, a molecule or a chemical compound. The analyte specifically may be an analyte that may be present in a bodily fluid or a body tissue. The term analyte specifically may encompass atoms, ions, molecules and macromolecules, in particular biological macromolecules such as nucleic acids, peptides and proteins, lipids, and metabolites. Further examples of potential analytes to be detected will be given in further detail below.

The term "concentration", as used herein, is a broad term and is to be given its ordinary and customary meaning to a person of ordinary skill in the art and is not to be limited to a special or customized meaning. The term specifically may refer, without limitation, to a numerical indication of an amount of a first entity within a second entity, specifically of the analyte in the sample. Said amount may generally be indicated in various ways known to the skilled person. The concentration may comprise at least one of a molar concentration, an equivalence concentration, a mass concentration, a volume concentration and a particle concentration. Further options as well as combinations are feasible. As an example, under ambient conditions, the sample may be a liquid sample, the analyte may be a solid analyte and the concentration may be defined via a ratio of the mass of the solid analyte per volume of the liquid sample, e.g. in g/L.

The term "sample" as used herein is a broad term and is to be given its ordinary and customary meaning to a person of ordinary skill in the art and is not to be limited to a special or customized meaning. The term specifically may refer, without limitation, to an arbitrary entity or aliquot of at least one material being subject of an analysis. Specifically, the sample may comprise at least one fluid, such as at least one liquid and/or at least one gas. The sample may be comprised in a defined or definable space or may be present in an open space such as in an open surrounding. The sample may be present in a static state or may flow continuously or discontinuously. The sample may, as an example, be a pure liquid or a homogeneous or heterogeneous mixture, such as a dispersion, an emulsion or a suspension. Additionally or alternatively, mixtures of gases or mixtures of gases with liquids or solids may be used. Specifically, the sample may contain atoms, ions, molecules and macromolecules, in particular biological macromolecules such as nucleic acids, peptides and proteins, lipids and metabolites, and also biological cells and cell fragments. In an embodiment not encompassed by the wording of the claims, samples can, however, also be or comprise calibration solutions, reference solutions, reagent solutions or solutions containing standardized analyte concentrations, so-called standards.

Typical samples to be analyzed may be bodily fluids. The term "bodily fluid" as used herein is a broad term and is to be given its ordinary and customary meaning to a person of ordinary skill in the art and is not to be limited to a special or customized meaning. The term specifically may refer, without limitation, to a fluid originating from at least one body, specifically from at least one human body. Specifically, the bodily fluid may comprise at least one aqueous solution. As an example, the bodily fluid may comprise at least one of blood, plasma, serum, urine, cerebrospinal fluid, lachrymal fluid, cell suspensions, cell supernatants, cell extracts, tissue lysates, saliva, tear fluid and interstitial fluid.

The method steps may be performed in the given order. It shall be noted, however, that a different order may also be possible. Further, one or more of the method steps may be performed once or repeatedly. Further, two or more of the method steps may be performed simultaneously or in a timely overlapping fashion. The method may comprise further method steps which are not listed.

The term "sensor device" as used herein is a broad term and is to be given its ordinary and customary meaning to a person of ordinary skill in the art and is not to be limited to a special or customized meaning. The term specifically may refer, without limitation, to a detector configured for at least one of qualitatively and quantitatively determining at least one property of another object or entity, such as configured for at least one of qualitatively and quantitatively analyzing at least one subject of investigation. Specifically, the sensor device may be configured for qualitatively and/or quantitatively detecting at least one analyte in the sample, specifically at a high degree of sensitivity. The sensor device specifically may be configured for determining the concentration of at least one analyte in the sample. The sensor device comprises at least one field effect transistor. The term "field effect transistor" as used herein is a broad term and is to be given its ordinary and customary meaning to a person of ordinary skill in the art and is not to be limited to a special or customized meaning. The term specifically may refer, without limitation, to a functional semiconductor element comprising at least one source electrode, at least one drain electrode and at least one gate electrode. The term "electrode" as used herein is a broad term and is to be given its ordinary and customary meaning to a person of ordinary skill in the art and is not to be limited to a special or customized meaning. The term specifically may refer, without limitation, to a functional element configured to perform a current measurement and/or a voltage measurement and/or configured to apply a current and/or an electrical potential and/or a voltage to an element in electrical contact with the electrode. In particular, the electrode may comprise a conducting and/or a semiconducting material. As an example, the electrode may comprise at least one metallic material and/or at least one organic or inorganic semiconducting material, having at least one conducting or semiconducting surface. The surface itself may form the electrode or a part of the electrode. As an example, the electrode may comprise at least one material, specifically at least one surface material, having an electrical conductivity of at least 1 S/m, e.g. at least 1000000 S/m, either isotropically or anisotropically in at least one direction. The source electrode, the drain electrode and the gate electrode of the field effect transistor may each be individually applicable with at least one predefined electrical potential. The potential may be a constant potential or a varying potential. Generally, a potential may be given with respect to ground. As an example, a gate potential V_{G} may given with respect to ground. However, as commonly used, the gate potential V_{G} may also refer to a potential difference between the source electrode and the gate electrode of the field effect transistor. A potential difference between two electrodes may generally be referred to as a voltage. As an example, a drain-source-voltage V_{DS} generally refers to a potential difference between the drain electrode and the source electrode of the field effect transistor. Analogously, a drain-source-current I_{DS} generally refers to a current between the drain electrode and the source electrode.

Generally, the field effect transistor further comprises at least one channel. The term "channel" as used herein is a broad term and is to be given its ordinary and customary meaning to a person of ordinary skill in the art and is not to be limited to a special or customized meaning. The term specifically may refer, without limitation, to a semiconducting component or a part thereof configured to conduct a current between the source electrode and the drain electrode. The channel may have at least one semiconducting material and/or at least one doped semiconducting material. The semiconducting material may be or may comprise at least one of an inorganic semiconducting material and an organic semiconducting material. Typically, a semiconducting material exhibits an electrical conductivity σ of 10⁻⁶ S/m < σ < 10⁶ S/m. In the field of organic semiconductors, however, due to the impact of the low charge carrier mobilities, due to the molecular orbitals and/or due to the low charge carrier densities, however, this description is often not fully applicable. Thus, organic conductive materials are often denoted as organic semiconductors, even though their conductivity may be higher than 10⁶ S/m, such as graphene, or lower than 10⁻⁶ S/m.

In particular, the semiconducting material may comprise one, two or more regions, preferably two to ten regions, more preferably three regions, wherein each region may be n-type doped or p-type doped. Specifically the semiconducting material may comprise an inorganic and/or organic semiconducting material. The channel may be able to conduct a current between the source electrode and the drain electrode only under specific external conditions. The conditions may include a temperature of the channel and/or the voltage or electrical potential applied to the channel either directly, e.g. via a surface of the channel, or via the gate electrode or via an external electrode. In particular, the channel may be constituted by at least one semiconducting material, such as by at least one semiconducting layer. As an example, inorganic and/or organic semiconducting materials may be used. The gate electrode may be in direct physical contact with the channel. In this configuration the field effect transistor may generally be referred to as "non-insulated-gate field effect transistor" (NIGFET). In particular, the gate electrode may be at least partially identical with the channel or with a surface of the channel. Alternatively, the gate electrode may be in indirect physical contact with the channel, e.g. by using one or more electrically insulating materials interposed in between the gate electrode and the channel. In this configuration the transistor may generally be referred to as "insulated-gate field effect transistor" (IGFET).

The insulated-gate field effect transistor specifically may be implemented as a "metal-insulator-semiconductor field effect transistor" (MISFET). The gate electrode may comprise at least one metal which may be insulated from the channel by at least one electrical insulating material. The channel may comprise at least one semiconducting material. Thus, the field effect transistor, specifically the MISFET, may comprise at least one insulating material also referred to as dielectric material or simply as dielectric. Specifically, the insulation of the gate electrode from the channel may be constituted by an oxide. In this configuration the field effect transistor may generally be referred to as "metal-oxide-semiconductor field effect transistor" (MOSFET). However, other materials for insulation of the gate electrode are feasible. The channel of the field effect transistor may be in physical contact with an electrolyte solution, which may constitute or form part of the gate electrode. An ionic double layer may be formed, that may serve as insulation of the gate electrode from the channel. In this configuration, the field effect transistor may be referred to as a "solution-gated or liquid-gated FET". The electrolyte solution may comprise substances that may influence the potential applied to the channel upon close proximity or adsorption to the channel and/or the insulation of the channel, thus allowing the detection of chemical species. In this configuration, the field effect transistor may be referred to as a "chemical field effect transistor" or ChemFET. In particular, a ChemFET may be configured for the detection of ionic species forming an "ion-sensitive field effect transistor" (ISFET) that may be sensitive to H⁺ and/or other ionic species. A layer sensitive to ionic species, such as Al₂O₃, Si₃N₄ or Ta₂O₅, may be in contact with the channel or may form part of the gate electrode of the ISFET and/or may form part of the channel and the gate electrode. In another configuration, the ChemFET may comprise a layer of immobilized enzymes as part of the gate electrode and/or the channel of the field effect transistor. In this configuration, the field effect transistor may be referred to as an "enzyme field effect transistor" (ENFET). Binding of the enzyme to the analyte may affect the potential applied to the channel and allow detection of the analyte. Thus, the ENFET is an example of a field effect transistor-based biosensor (BioFET). As a BioFET the field effect transistor may comprise a layer of immobilized biomolecules as biorecognition elements able to bind one or more species of molecules, specifically biomolecules, where the binding reaction may either directly or indirectly affect the potential applied to the channel.

The field effect transistor may further be implemented as an "extended gate field effect transistor" (EGFET). The term "extended gate field effect transistor" as used herein is a broad term and is to be given its ordinary and customary meaning to a person of ordinary skill in the art and is not to be limited to a special or customized meaning. The term specifically may refer, without limitation, to a field effect transistor comprising a gate electrode configured to allow a spatial separation of the channel of the field effect transistor from a process or reaction that sets or affects the potential of the gate electrode. Such an electrode may generally be referred to as an "extended gate electrode". Thus, the gate electrode, as an example, may comprise at least one portion being in contact with the material to be sensed or with the sample, and, optionally, at least one portion being in close proximity to the channel, e.g. by being separated from the channel only by at least one layer of insulating material, wherein the portions are electrically connected. Thus, the extended gate electrode of an extended gate field effect transistor may allow to physically separate the process of applying a potential to the channel and the process of applying a potential to the gate electrode.

The field effect transistor may comprise at least one substrate. The substrate may have purely mechanical properties and function, such as for carrying the above-mentioned components of the field effect transistor. Alternatively, however, the substrate may also be fully or partially identical with one or more of the above-mentioned components. Thus, as an example, the at least one channel may fully or partially be embodied within the substrate.

The sensor device further comprises at least one sensing electrode. The term "sensing electrode" as used herein is a broad term and is to be given its ordinary and customary meaning to a person of ordinary skill in the art and is not to be limited to a special or customized meaning. The term specifically may refer, without limitation, to an electrode configured for being exposed to the sample with the analyte. The sensing electrode may specifically be functionalized for interacting with the analyte as will be described in further detail below. As indicated, the sensing electrode is electrically connected to the gate electrode. In this case, direct contact between the sample and the gate electrode may be avoided. Additionally or alternatively, the sensing electrode is integrated or incorporated into the gate electrode. Thus, the gate electrode may be or may comprise the sensing electrode. Specifically, the sensing electrode may be an extended gate electrode of an EGFET. In any case, a voltage applied to the sensing electrode may be transferred to the gate electrode. However, the electrical potential applied to the sensing electrode may be affected by the analyte, specifically for the sensing electrode being functionalized with regard to the analyte. The electrical potential applied to the sensing electrode may be applied to the sensing electrode via the sample, specifically by using at least one electrolyte, more specifically at least one electrolyte solution. Thus, a concentration of the analyte in the sample may affect a potential applied to the gate electrode and consequently an electrical field seen by the channel of the field effect transistor. As an example, the analyte may comprise ions affecting the electrical field, e.g. by shielding an externally applied electrical field. Thus, the concentration of the analyte may eventually affect the electrical response of the field effect transistor, e.g. a drain-source-current I_{DS} of the field effect transistor generated at a specific drain-source-voltage V_{DS}.

The sensor device further comprises at least one control device. The term "control device" as used herein is a broad term and is to be given its ordinary and customary meaning to a person of ordinary skill in the art and is not to be limited to a special or customized meaning. The term specifically may refer, without limitation, to an arbitrary electronic device configured for at least one of monitoring, regulating and controlling one or more operations of the field effect transistor. The control device may be configured for controlling or setting at least one voltage at an electrode of the field effect transistor. The control device may be configured for measuring at least one current between two electrodes of the field effect transistor. The control device may be configured for controlling or setting at least one current between two electrodes of the field effect transistor. The control device may be configured for measuring at least one voltage at an electrode of the field effect transistor. Combinations of the indicated options may be feasible. The control device may comprise at least one source measure unit (SMU). The control device may comprise at least one power supply. The control device may comprise at least one of a multimeter, a voltmeter, an amperemeter and an ohmmeter.

The control device may specifically be or may comprise at least one galvanostat also known as amperostat. Thus, the control device may be configured for keeping at least one current constant, specifically the drain-source current I_{DS}, while measuring at least one voltage, specifically the drain-source voltage V_{DS}. Thus, the field effect transistor, which may as said specifically be a MOSFET, may be connected to or connectable to the galvanostat, e.g. by using at least one wired connection. More specifically, at least the source electrode and the drain electrode of the field effect transistor may be connectable to the galvanostat. The control device may comprise at least one ground. The galvanostat may be grounded. The control device may comprise at least one output, specifically at least one analog output, wherein the analog output may be configured for applying at least one potential, specifically the gate potential V_{G}. The analog output may be grounded. Thus, the gate potential V_{G} may be applied relative to ground. The control device may also comprise at least one digital output and/or interface, e.g. for communication with at least one external device and/or user.

The control device may comprise at least one processing unit. The term "processing unit" as used herein is a broad term and is to be given its ordinary and customary meaning to a person of ordinary skill in the art and is not to be limited to a special or customized meaning. The term specifically may refer, without limitation, to an arbitrary device adapted to perform the named operations, preferably by using at least one data processing device and, more preferably, by using at least one processor and/or at least one application-specific integrated circuit. As an example, the processing unit may comprise at least one data processing device having a software code stored thereon comprising a number of computer commands. The processing unit may provide one or more hardware elements for performing one or more of the named operations and/or may provide one or more processors with software running thereon for performing one or more of the named operations. As an example, the processing unit may comprise one or more programmable devices such as one or more computers, application-specific integrated circuits (ASICs), Digital Signal Processors (DSPs), or Field Programmable Gate Arrays (FPGAs). Additionally or alternatively, however, the processing unit may also fully or partially be embodied by hardware. The processing unit may specifically be configured for performing at least one measurement cycle. An information as determined by the processing unit may be provided to at least one of a further apparatus and/or to a user, specifically in at least one of an electronic, visual, acoustic, or tactile fashion. The information as determined by the processing unit may be stored in a memory storage and/or in a separate storage device and/or may be passed on via at least one interface, such as a wireless interface and/or a wire-bound interface. The processing unit may further be configured for controlling at least a portion of the sensing device, specifically at least a portion of the control device.

The control device is configured for applying the operation parameters to the field effect transistor. The term "operation parameter" as used herein is a broad term and is to be given its ordinary and customary meaning to a person of ordinary skill in the art and is not to be limited to a special or customized meaning. The term specifically may refer, without limitation, to a parameter, specifically an electrical parameter, which is characteristic for a specific operation of a device, specifically of the field effect transistor. In other words, the operation parameters may define the operation of the field effect transistor. Specifically, the operation parameters may define and/or specify at least one of: which voltages and/or potentials are applied to which electrodes of the field effect transistor; which currents are applied to which electrodes of the field effect transistor; which voltages and/or potentials are measured between which electrodes of the field effect transistor; which currents are measured between which electrodes of the field effect transistors. Further options as well as combinations are feasible. The operation parameters may generally both refer to a type of a parameter, e.g. a voltage or a current, and a value of the parameter, e.g. 1 V or 1 A. Further details and embodiments will be outlined below. The operation parameters may additionally or alternatively refer to a shape of a parameter. As indicated, the operation parameters may for example comprise a voltage or a current. As will also be outlined in further detail below, the voltage or the current may for example be AC or DC or comprise pulses or periodic signals which may define the shape of the voltage or the current. The operation parameters referring to the shape of a parameter may be, without limitation, e.g. a frequency or a pulse time.

The control device is configured for monitoring at least one signal value with the field effect transistor. The term "signal" as used herein is a broad term and is to be given its ordinary and customary meaning to a person of ordinary skill in the art and is not to be limited to a special or customized meaning. The term specifically may refer, without limitation, to an entity or function used for carrying information. Specifically, the signal may comprise at least one electrical signal. More specifically, the signal may comprise at least one of a voltage and a current. Thus, the signal may comprise at least one analog signal. Additionally or alternatively, the signal may comprise at least one digital signal, e.g. a processed or at least preprocessed signal. The signal may vary over time. The signal may comprise at least one AC signal. Additionally or alternatively, the signal may comprise at least one DC signal. Above and in the following, AC refers to alternating current and DC refers to direct current as generally used by the skilled person. The signal may respond, specifically electrically, to an observable change in an environment influencing the signal. As an example, the signal may be the drain-source-current I_{DS} of the field effect transistor which may be affected by an applied drain-source-current V_{DS} and an applied gate potential V_{G} plus a presence of an analyte affecting an actual electrical field seen by the channel of the field effect transistor.

The term "signal value" as used herein is a broad term and is to be given its ordinary and customary meaning to a person of ordinary skill in the art and is not to be limited to a special or customized meaning. The term specifically may refer, without limitation, to a value describing a measurable property of the signal, such as an intensity of the signal. As said, the signal may specifically comprise at least one electrical signal, more specifically at least one of a voltage and a current. As further said, the signal may comprise at least one DC signal. Thus, the signal value may comprise at least one of a voltage value and a current value also referred to as current strength. As also said, the signal may comprise at least one AC signal. Thus, the signal value may comprise at least one of an amplitude, a mean value such as a DC bias, a frequency, a phase, a phase difference and a duty cycle. Generally, the signal may be subject to noise, e.g. 1/f noise, as the skilled person will know. The noise may distort the monitored signal values and thus deteriorate the reliability of a measurement. The noise may specifically also depend on the environment influencing the signal. Thus, as an example, it may be expedient to find operation parameters leading to as little noise as possible.

The parameter selection step, step ii., comprises performing a plurality of evaluation measurements with the field effect transistor by using various sets of operation parameter candidates and by selecting the set of operation parameters in accordance with at least one optimization criterion monitored during the evaluation measurements. The term "evaluation measurement" as used herein is a broad term and is to be given its ordinary and customary meaning to a person of ordinary skill in the art and is not to be limited to a special or customized meaning. The term specifically may refer, without limitation, to a measurement or premeasurement performed for finding an adequate or optimal measurement setup for a subsequently performed measurement of actual interest. Specifically, the evaluation measurements may be premeasurements performed for finding best operation parameters for subsequently determining the concentration of the analyte in the sample. As an example, the evaluation measurements may be used for finding operation parameters producing as little noise as possible as indicated above.

The term "operation parameter candidate" as used herein is a broad term and is to be given its ordinary and customary meaning to a person of ordinary skill in the art and is not to be limited to a special or customized meaning. The term specifically may refer, without limitation, to an operation parameter applied during the evaluation measurements. Thus, the operation parameter candidate may be a possible operation parameter for determining the concentration of the analyte, which possible operation parameter is to be tested beforehand in at least one evaluation measurement. Specifically, as indicated, the operation parameter candidates may be applied to the field effect transistor in sets. In other words, one or more operation parameter candidates may be applied to the field effect transistor together, specifically simultaneously, as a set. As an example, a specific drain-source-voltage V_{DS} combined with a specific a gate potential V_{G} may be applied to the field effect transistor and a corresponding drain-source-current I_{DS} may be measured in an evaluation measurement. Further in this example, such a procedure may be repeated for a number of sets of specific drain-source-voltages V_{DS} and gate potentials V_{G} and eventually the specific set may be selected for determining the concentration of the analyte which yields e.g. the least noise in the measured drain-source-current I_{DS}. A plurality of further options are feasible and further embodiments will be outlined below.

The term "optimization criterion" as used herein is a broad term and is to be given its ordinary and customary meaning to a person of ordinary skill in the art and is not to be limited to a special or customized meaning. The term specifically may refer, without limitation, to a criterion which is used for optimizing at least one process, specifically at least one measurement such as determining the concentration of the analyte in the sample. The optimization criterion, as an example, may be or may comprise at least one target condition which is to be fulfilled as far as possible. For optimization purposes, deviations from the fulfillment of the target condition may be used. Specifically, one or more sets of operation parameter candidates may be compared according to the optimization criterion and the specific set may be selected which yields the best result according to the optimization criterion. Thus, at least one measurable value may be determined which, for each set of operation parameters, describes a deviation of a result which is achieved by using the set of operation parameters from a target result which is defined by the at least one target criterion, and the measurable value may be used for selecting the optimum set of operation parameters.

The term "monitoring" including any grammatical variation thereof as used herein is a broad term and is to be given its ordinary and customary meaning to a person of ordinary skill in the art and is not to be limited to a special or customized meaning. The term specifically may refer, without limitation, to an action of at least one of measuring, tracking, tracing, observing and recording at least one entity, e.g. the optimization criterion as indicated above, specifically over a period of time, such as continuously or repeatedly over the at least one period of time. Specifically, monitoring the optimization criterion during the evaluation measurements may comprise evaluating the optimization criterion for at least one operation parameter candidate. Thus, it may be possible to compare different operation parameter candidates by monitoring the optimization criterion. As an example, the optimization parameter may be a signal-to-noise-ration (SNR), which may be determined for different operation parameter candidates such as for example specific gate potentials V_{G} and/or specific drain-source-voltages V_{DS}. By monitoring the signal-to-noise-ration (SNR), the operation parameter candidates yielding the highest signal-to-noise-ration (SNR) may for example be identified.

Thereby, determining the concentration of the analyte in the sample may be optimized according to the optimization criterion. Obviously, a different choice of the optimization criterion may lead to a different set of operation parameter candidates being selected as operation parameters. As an example and as already indicated, the optimization criterion may relate to a signal noise, such that a set of optimization parameters may be selected which in comparison leads to the lowest signal noise. Further details and embodiments will be outlined below.

The measurement step, step iii., comprises detecting the concentration of the analyte by applying the set of operation parameters selected in step ii. to the field effect transistor and by determining at least one signal value with the field effect transistor. Specifically, selected potentials, voltages and/or currents may be applied to selected electrodes of the field effect transistor. As an example, as already indicated, a selected combination of a drain-source-voltage V_{DS} and gate potential V_{G} may applied to the field effect transistor and a corresponding drain-source-current I_{DS} may be measured as a signal value. As said, further options are feasible and further embodiments will be outlined below. Based on the determined signal value, the concentration of the analyte may be determined by using at least one known relation between the determined signal value and the concentration, e.g. a look-up table or a transfer function. The relation may for instance be determined in at least one calibration, specifically by using at least one calibrator. Further details will be given below.

The set of operation parameters selected in step ii. may comprise at least two operation parameters selected from the group consisting of: a gate potential V_{G} of the field effect transistor; a drain-source-current I_{DS} of the field effect transistor. The gate potential V_{G} may be applied in various forms. The gate potential V_{G} may be applied as a constant potential or as a varying potential, wherein the varying potential may for instance comprise predefined DC levels or an AC voltage having a predefined frequency. Further, galvanostatic parameters such as an acquisition rate, pulses or steps may be varied. Thus, using a specific voltage or current as operation parameter may comprise using specifically at least one of a magnitude, a phase and a frequency of the voltage or the current as the operation parameter. Generally, at least one characteristic of the voltage or the current may be used as the operation parameter.

The sensor device, such as the control device, may be configured for applying a gate potential V_{G} to the gate electrode of the field effect transistor via the sample and the sensing electrode and further for monitoring a drain-source-voltage V_{DS} required for achieving a predefined drain-source-current I_{DS}. Thus, the drain-source-voltage V_{DS} may specifically be the signal value determined in step iii. from which the concentration of the analyte is derived. As said, the control device may comprise at least one analog output. As an example, the control device and specifically the analog output may apply the gate potential V_{G} to the sample, e.g. by using at least one electrode connected to the sample. As an example, the sample may comprise an electrolyte solution and the electrode may be inserted into the electrolyte solution. Further options may be feasible. As already indicated, the sensing electrode may specifically be exposed to the sample and functionalized with regard to the analyte. Thus, the applied gate potential V_{G} may be affected by the sample and specifically by the analyte. Specifically, as already indicated, the corresponding electric field in the channel of the field affect transistor may be affected by the analyte. Thus, the induced drain-source-current I_{DS} may be affected by the analyte. As said, the drain-source-current I_{DS}, being a selected operation parameter, may be predefined. Thus, the drain-source-current I_{DS} may be kept in its predefined state, i.e. constant, by using the control device. In other words, the control device, which may as said specifically comprise a galvanostat, may be configured for maintaining the predefined drain-source-current I_{DS} as selected in step ii.. This may specifically be accomplished by accordingly varying the drain-source-voltage V_{DS} as the skilled person will understand. In other words, the control device may be configured for readjusting or regulating the drain-source-current I_{DS} by varying the drain-source-voltage V_{DS}. As said, the control device may further be configured for monitoring the drain-source-voltage V_{DS} required for achieving the predefined drain-source-current I_{DS}. As the skilled person will also understand in the light of the description given above, by monitoring the conducted changes in the drain-source-voltage V_{DS}, the concentration of the analyte can be derived.

The control device may comprise at least one feedback loop for controlling the drain-source-voltage V_{DS} required for achieving the drain-source-current I_{DS}. The term "feedback loop" as used herein is a broad term and is to be given its ordinary and customary meaning to a person of ordinary skill in the art and is not to be limited to a special or customized meaning. The term specifically may refer, without limitation, to an entity, specifically an electrical circuit or a portion of an electrical circuit, configured for feeding a signal response generated by using an input signal and a system back to the input signal, and/or by coupling a signal or a quantity derived from the signal back to an origin of the signal. As said, the drain-source-current I_{DS} may be affected by the analyte in the sample, but may still be maintained as predefined by the control device. The feedback loop may be configured for measuring the drain-source-current I_{DS}, specifically the actual drain-source-current I_{DS} as affected by the analyte, and further for coupling the measured drain-source-current I_{DS} back to the control device. Thus, the control device may be configured for determining and applying a drain-source-voltage V_{DS} required for again achieving the predefined drain-source-current I_{DS} by using the feedback loop.

The set of operation parameters selected in step ii. may comprise a gate potential V_{G} of the field effect transistor to be applied to the sample and a drain-source-current I_{DS} of the field effect transistor to be applied to the field effect transistor during detecting the concentration of the analyte. Step iii. may comprise determining a drain-source-voltage V_{DS} required for achieving the drain-source-current I_{DS} as the signal value and may further comprise deriving the concentration of the analyte from the drain-source-voltage V_{DS}. Analog to above, based on the determined drain-source-voltage V_{DS} as the signal value, the concentration of the analyte may be determined by using at least one known relation between the determined drain-source-voltage V_{DS} and the concentration, e.g. a look-up table or a transfer function. The known relation may for instance be determined in at least one calibration, specifically by using at least one calibrator.

The optimization criterion may be related to at least one measurable optimization criterion value. Specifically, the optimization criterion value may be quantifiable and/or comparable. The optimization criterion value may be selected from the group consisting of: a signal-to-noise-ratio SNR; a signal intensity; a signal noise; a signal drift. As already indicated, a low signal noise may for instance facilitate reliable measurements. As the skilled person will know, in this respect, the relation between the signal intensity and the signal noise may specially be decisive, which may generally be addressed by the signal-to-noise-ratio SNR, i.e. the quotient of signal intensity over noise intensity. As an example, more noise may be acceptable for reliable measurements if the signal intensity is still considerably higher in comparison. As the skilled person will further know, a signal may drift over time for various reasons such as varying ambient conditions or a variation of at least one component within the sensor device, specifically within the field effect transistor, e.g. due to a material degradation. Such a drift, similar to the above-mentioned noise, may also have an effect on the monitored signal values and thus on the reliability of a measurement.

The set of operation parameters in step ii. may be selected by selecting operation parameters of an evaluation measurement resulting in one of a maximum optimization criterion value and a minimum optimization criterion value monitored during the evaluation measurements. As an example, the set of operation parameters in step ii. may be selected by selecting operation parameters of an evaluation measurement resulting in at least one of a maximum signal-to-noise-ratio SNR, a maximum signal intensity, a minimum signal noise and a minimum signal drift. Specifically, the set of operation parameters in step ii. may be selected by selecting operation parameters of an evaluation measurement resulting in a maximum signal-to-noise-ratio SNR. Such a selection may facilitate precise and reliable measurements, specifically a precise and reliable determination of the concentration of the analyte in the sample. In such way, the most suitable operation parameters may be found for each specific measurement by using resource-saving and quickly adaptable measures. Specifically, a rather complex changing of hardware components may be avoided.

Performing the evaluation measurements in step ii. may comprise subsequently performing individual evaluation measurements. Each evaluation measurement may comprise varying one operation parameter while keeping further operation parameters constant. In such way, an effect of varying the operation parameter may be tracked in an isolated fashion and thus more reliably. As already outlined, the gate potential V_{G} and the drain-source-current I_{DS} of the field effect transistor may specifically be used as operation parameters and may be applied to the field effect transistor in a combined fashion within one set during an evaluation measurement. At least one evaluation measurement may comprise varying the gate potential V_{G} of the field effect transistor, specifically while keeping the drain-source-current I_{DS} of the field effect transistor constant. Thus, an effect of varying the gate potential V_{G} may be investigated and an optimal gate potential V_{G} may be determined. Additionally or alternatively, at least one evaluation measurement may comprise varying a drain-source-current I_{DS} of the field effect transistor, specifically while keeping a gate potential V_{G} of the field effect transistor constant. Thus, an effect of varying the drain-source-current I_{DS} may be investigated and an optimal drain-source-current I_{DS} may be determined. Eventually, the determined optimal gate potential V_{G} and the determined optimal drain-source-current I_{DS} may be applied to the field effect transistor for determining the concentration of the analyte in the sample. Further kinds of evaluation measurements may be feasible and an order of the evaluation measurements may be variable.

Generally, step ii. comprises selecting a first operation parameter for step iii. by performing at least one first evaluation measurement comprising varying the first operation parameter while keeping further operation parameters constant. Step ii. further comprises keeping the selected first operation parameter constant in at least one further evaluation measurement performed for selecting at least one further operation parameter for step iii. Generally, the terms "first", "second" and, if applicable, further numberings are merely used herein as nomenclature, without indicating an order or ranking. As indicated, the first operation parameter may comprise the gate potential V_{G} of the field effect transistor and the further operation parameter may comprise the drain-source-current I_{DS} of the field effect transistor.

Step ii. comprises performing the evaluation measurements under experimental conditions corresponding to step iii.. Specifically, step ii. comprises performing the evaluation measurements by using the same sample as in step iii. Additionally, step ii. may comprise performing the evaluation measurements under the same ambient conditions, e.g. temperature or humidity, as in step iii. Further, step ii. may comprise performing the evaluation measurements by using the same setup and/or settings for the sensing device, specifically for the control device. Specifically, step ii. may comprise performing the evaluation measurements by using the same measurement range as in step iii..

Step ii. may be re-performed when changing a measurement range in step iii.. The term "measurement range" as used herein is a broad term and is to be given its ordinary and customary meaning to a person of ordinary skill in the art and is not to be limited to a special or customized meaning. The term specifically may refer, without limitation, to at least one interval over which a measurement is conducted. The measurement range may comprise a starting value and a final value and/or a minimum value and a maximum value, defining the interval. As the skilled person will already know, specifically with respect to measurements involving field effect transistors, at least one entity, e.g. the gate potential V_{G}, may be swept through a chosen measurement range during a measurement such as when recording a transfer curve of the field effect transistor. **In** this example, the gate potential V_{G} may be varied, e.g. continuously or by using discrete levels, from a starting value to a final value, which define the measurement range of gate potential V_{G} in this case. Analog considerations may apply mutatis mutandis to other field effect transistor parameters and to other parameters in general as the skilled person will understand. The measurement ranges may affect the performance of the field effect transistor and specifically the performance of the field effect transistor for a selected set of operation parameters. In other words, different operation parameters may be more suitable for a different measurement range. Thus, when changing the measurement range in step iii., it may facilitate precise and reliable measurements to re-perform step ii. and, at least in some cases, to select an adapted new set of operation parameters.

Step ii. may further comprise performing at least one calibration determining a relation between the concentration of the analyte and the signal value of the field effect transistor. The term "relation" as used herein is a broad term and is to be given its ordinary and customary meaning to a person of ordinary skill in the art and is not to be limited to a special or customized meaning. The term specifically may refer, without limitation, to a connection or assignment between two entities. Specifically, a signal value may be measured by using the sensor device and the relation may assign the measured signal value to a specific concentration of the analyte. The relation may comprise at least one of a look-up table, a transfer function, an algorithm and a model. Thus, the relation may, e.g. be determined by providing a plurality of test samples having a known concentration of the analyte, and may further comprise recording the corresponding signal values of the field effect transistor for said test samples. Thereafter, a relationship, such as a calibration curve, may be determined, e.g. by regression. The relation, thus, may comprise at least one trained model, such as a model trained by regression or other methods known to the person skilled in the art. The relation specifically may be determined by performing a calibration before the actual measurement of interest.

The term "calibration" as used herein is a broad term and is to be given its ordinary and customary meaning to a person of ordinary skill in the art and is not to be limited to a special or customized meaning. The term specifically may refer, without limitation, to a process of comparing a measured value to a known standard, i.e. an entity having at least one precisely known property. The calibration may comprise measuring the known standard and assigning the measured value to the known property. Specifically, as will also be outlined in further detail below, the calibration may comprise determining at least one signal value with the field effect transistor for a sample with a known concentration of the analyte and further assigning the determined signal value to the known concentration.

The calibration may comprise determining a sensitivity of the sensor device. The term "sensitivity" as used herein is a broad term and is to be given its ordinary and customary meaning to a person of ordinary skill in the art and is not to be limited to a special or customized meaning. The term specifically may refer, without limitation, to a ratio of a change of an output value in relation to a change of a corresponding input value. Specifically, the sensitivity may describe a change in a signal value as determined by using the sensing device in relation to a change in the concentration of the analyte. More specifically, the sensitivity may describe a change in a determined drain-source-voltage V_{DS} for a given change in the concentration of the analyte, i.e. a ratio of voltage change per concentration change.

The calibration may comprise using at least one calibrator, specifically a set comprising a plurality of different calibrators. The term "calibrator" as used herein is a broad term and is to be given its ordinary and customary meaning to a person of ordinary skill in the art and is not to be limited to a special or customized meaning. The term specifically may refer, without limitation, to a known standard, specifically a sample having known properties. More specifically, the calibrator may comprise a sample having a known concentration of the analyte. Thus, by analyzing the calibrator having a known concentration of the analyte, the electrical response of the sensor device to the analyte may be assigned to a corresponding concentration of the analyte as already outlined above.

As further already indicated, the field effect transistor may be selected from the group consisting of: an extended gate field effect transistor (EGFET); an ion sensitive field effect transistor (ISFET); a chemically sensitive field effect transistor (ChemFET); a biological field effect transistor (BioFET); an enzyme field effect transistor (ENFET); a solution- or liquid-gated field effect transistor; a graphene based field effect transistor. Specifically, the field effect transistor may be an extended gate field effect transistor (EGFET). The sensing electrode may be an extended gate electrode. The sensing electrode may comprise at least one functional component on its surface. The functional component may, as an example, comprise at least one chemical compound deposited on at least one surface of the sensing electrode, such as on a metal surface or an electrically conducting carbon surface of the sensing electrode. The functional component may, as an example, be chemically linked to the sensing electrode, such as by at least one of a covalent bond, a complex bond, an ionic bond, a hydrogen bond, a dipole bond, a Van-der-Waals linkage. The functional component may be configured for, directly and/or indirectly, interacting with the analyte. The term "functional component" as used herein is a broad term and is to be given its ordinary and customary meaning to a person of ordinary skill in the art and is not to be limited to a special or customized meaning. The term specifically may refer, without limitation, to a material, such as a chemical compound, a molecule, a chemical blend or mixture, which specifically may, by itself, be electrically insulating, which may, however, form an electrostatic link between the analyte and the sensing electrode, such that an electrostatic potential of the sensing electrode is influenced, via the functional component, by the presence or absence of the analyte. Thus, the functional component may comprise spacer molecules which, as an example, may be bonded to the surface of the sensing electrode, e.g. by one or more of the above-identified linkages, and which may comprise at least one bonding site for linkage to the analyte to be detected. The spacer molecules may be configured such that the electrostatic potential of the sensing electrode differs between the case in which no analyte is present and the case in which the analyte is present. The functional component may be capable of binding the analyte, such as by at least one of a covalent bond, a complex bond, an ionic bond, a hydrogen bond, a dipole bond, a Van-der-Waals linkage. For this purpose, the functional component may comprise at least one binding site. For further information regarding the functional component reference may be made to pages 1 to 14 and specifically to page 8 of Gründler, Peter. Chemical sensors: An introduction for scientists and engineers. Springer Science & Business Media, 2007. The functional component may comprise at least one ionophore, specifically valinomycine. Additionally or alternatively, the functional component may comprise at least one receptor compound. The receptor compound may be capable of interacting with the at least one analyte, specifically of binding the at least one analyte, e.g. by one or more of the above-mentioned chemical linkages. The receptor compound may specifically be capable of binding the at least one analyte selected from the group consisting of: antibodies and fragments thereof, aptamers, peptides, enzymes, nucleic acids, receptor proteins or binding domains thereof.

The analyte may be selected from the group consisting of: potassium; sodium. Generally, any analyte configured for affecting a surface charge density, specifically of the sensing electrode, may be feasible. This may further generally include proteins, DNA and electrolytes. The sample may comprise a bodily fluid, specifically at least one of blood, plasma, serum, urine, cerebrospinal fluid, lachrymal fluid, cell suspensions, cell supernatants, cell extracts, tissue lysates, saliva, tear fluid and interstitial fluid.

The method specifically may be at least partially computer-implemented, specifically at least one of steps ii. and iii.. Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing samples and/or certain aspects of performing actual measurements.

Further disclosed and proposed herein is a computer program (not according to claimed invention) comprising instructions which, when the program is executed by the sensor device according to any one of the embodiments disclosed above or below in further detail referring to a sensor device, cause the sensor device to perform at least one of steps ii. and iii. of the method according to any one of the embodiments disclosed above or below in further detail referring to a method. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one or more of method steps ii. and iii. as indicated above may be performed by using a computer or a computer network, preferably by using a computer program.

Further disclosed and proposed herein is a computer program product (not in accordance with claimed invention) having program code means, in order to perform the method according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier (not in accordance with claimed invention) having a data structure stored thereon, which, after loading into a computer or computer network, such as into a working memory or main memory of the computer or computer network, may execute the method according to one or more of the embodiments disclosed herein.

Further disclosed and proposed herein is a computer program product (not in accordance with claimed invention) with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Further disclosed and proposed herein is a modulated data signal (not in accordance with claimed invention) which contains instructions readable by a computer system or computer network, for performing the method according to one or more of the embodiments disclosed herein.

Further disclosed and proposed herein is a computer-readable storage medium (not in accordance with claimed invention) comprising instructions which, when the program is executed by the sensor device according to any one of the embodiments disclosed above or below in further detail referring to a sensor device cause the sensor device to perform at least one of steps ii. and iii. of the method according to any one of the embodiments disclosed above or below in further detail referring to a method.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

The methods according to the present invention may provide a large number of advantages over known methods.

In particular, they may allow to efficiently determine an optimal measurement setup for determining a concentration of an analyte in a sample. Specifically, they may allow to quickly find optimal operation parameters for determining the concentration of the analyte with high performance and reproducibility. By doing so, changing hardware components, which may typically be a rather complex process, may be avoided. In case changing hardware components still becomes necessary, e.g. due to an end of a service life of a hardware component, adapting to the new component may be accomplished efficiently. Thus, the methods according to the present invention may be flexibly adaptable and widely applicable. Further, they may specifically allow for high time resolution measurements, efficient multiplexing of different sensor devices and efficient data processing.

The term "multiplexing" as used herein is a broad term and is to be given its ordinary and customary meaning to a person of ordinary skill in the art and is not to be limited to a special or customized meaning. The term specifically may refer, without limitation, to a simultaneous detection of multiple analytes from a single sample. The term specifically may refer, without limitation, to methods for simultaneous on-site detection of different analytes from a single specimen. The term specifically may refer, without limitation, to an action of combining a plurality of signals and transferring them via one shared medium, such as simultaneously or in an alternating fashion. Specifically, one gate potential V_{g} may be transferred via one sample to a plurality of field effect transistors. The sensor device may comprise a plurality of sensing electrodes exposed to the sample. The sensing electrodes may for example each be connected to one field effect transistor. Different sensing electrodes may be configured for measuring different analytes in the sample. As an example, a first sensing electrode may be configured for measuring potassium, a second sensing electrode may be configured for measuring magnesium and a third sensing electrode may be configured for measuring chloride. The sensing electrode may specifically be in contact with one liquid sample via which a gate potential V_{g} may be applied to the gate electrodes of the field effect transistors. Thus, the same gate potential V_{g} may be applied to all field effect transistors. For each field effect transistor, an individual drain-source-current I_{DS} may specifically be determined as an optimal operating parameter for determining the concentration of the analyte. Thus, when determining the concentration of one analyte, the determined individual drain-source-current I_{DS} of the corresponding field effect transistor may be monitored over time while keeping the gate potential V_{g} constant for all of the field effect transistors simultaneously.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is defined by the appended claims.

The embodiments are schematically depicted in the Figures. Therein, identical reference numbers in these Figures refer to identical or functionally comparable elements.

In the Figures:
- Figure 1: shows an exemplary sensor device for determining the concentration of at least one analyte in a sample in a schematic view;
- Figure 2: shows a flow chart of an exemplary embodiment of a method of determining the concentration of at least one analyte in a sample;
- Figures 3A-3C: show flow charts of exemplary embodiments of a calibration of a sensor device for determining the concentration of at least one analyte in a sample; and
- Figures 4A-4E: show experimental results of evaluation measurements of an exemplary embodiment of step ii. of a method of determining the concentration of at least one analyte in a sample plus a corresponding concentration detection of potassium.

### Detailed description of the embodiments

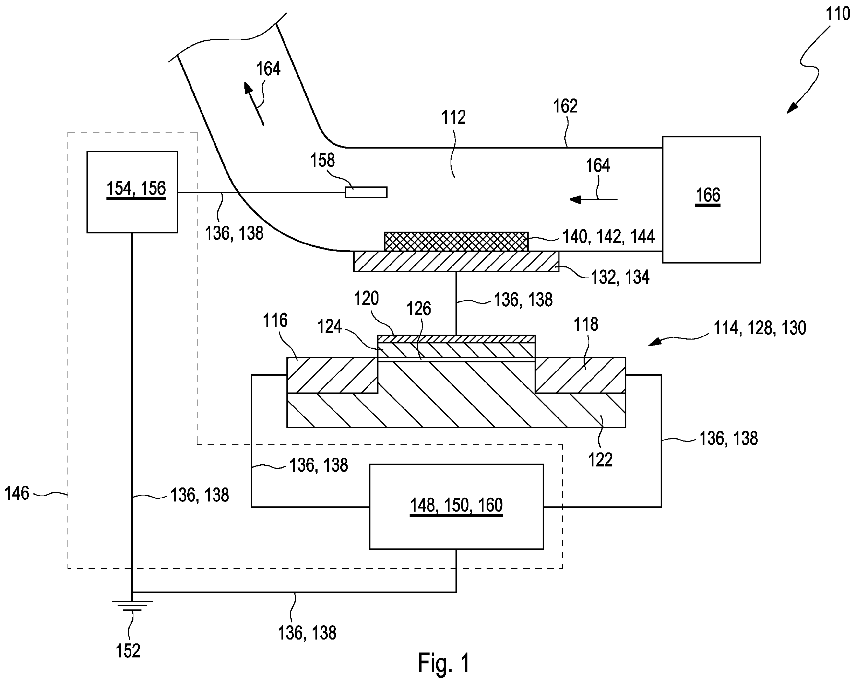

Figure 1 shows an exemplary sensor device 110 for determining the concentration of at least one analyte in a sample 112 in a schematic view. The sample 112 may specifically comprise a bodily fluid, specifically at least one of blood, interstitial fluid, plasma, saliva, urine, tear fluid. The analyte specifically may be selected from the group consisting of potassium and sodium. However, generally, other analytes and/or samples are also possible, specifically any analyte configured for affecting a charge density may be feasible. This may further generally include at least one of proteins, DNA and electrolytes.

The sensor device 110 comprises at least one field effect transistor 114. The field effect transistor 114 has at least one source electrode 116, at least one drain electrode 118 and at least one gate electrode 120. Further, the field-effect transistor 114 may comprise at least one semiconductor 122 and at least one dielectric 124, such as at least one layer of at least one solid dielectric medium. At an interface between the semiconductor 122 and the dielectric 124, a conductive channel 126 may form in the field effect transistor 114. Specifically, the field effect transistor 114 may be a metal-oxide-semiconductor field effect transistor (MOSFET) 128. Thus, the dielectric 124 may specifically be or may comprise at least one electrically isolating oxide. Generally, as an example, the field effect transistor 114 may be selected from the group consisting of: an extended gate field effect transistor (EGFET) 130; an ion sensitive field effect transistor (ISFET); a chemically sensitive field effect transistor (ChemFET); a biological field effect transistor (BioFET); an enzyme field effect transistor (ENFET); a solution- or liquid-gated field effect transistor, a graphene based field effect transistor.

The sensor device 110 further comprises at least one sensing electrode 132. The sensing electrode 132 is configured for being in contact with the sample 112. The sensing electrode 132 is at least one of electrically connected to the gate electrode 120 of the field effect transistor 114 and integrated into the gate electrode 120 of the field effect transistor 114. As indicated, the field effect transistor 114 may be an EGFET 130. The sensing electrode 132 may be an extended gate electrode 134. Thus, as shown in Figure 1, the sensing electrode 132 being an extended gate electrode 134 may specifically be electrically connected to the gate electrode 120, specifically by using at least one wire 136 and/or at least one electrically conducting trace 138.

The sensing electrode 132 may comprise at least one functional component 140 on its surface. The functional component 140 may be configured for interacting with the analyte. The functional component 140 may comprise at least one receptor compound 142. The receptor compound 142, generally, in this example or other examples, may be capable of interacting with the analyte, specifically of binding the analyte, such as by at least one of a covalent bond, a complex bond, an ionic bond, a hydrogen bond, a dipole bond, a Van-der-Waals linkage. The receptor compound 142 may specifically be capable of binding the at least one analyte selected from the group consisting of: antibodies and fragments thereof, aptamers, peptides, enzymes, nucleic acids, receptor proteins and binding domains thereof. Additionally or alternatively, the functional component 140 may comprise at least one ionophore 144, specifically valinomycine.

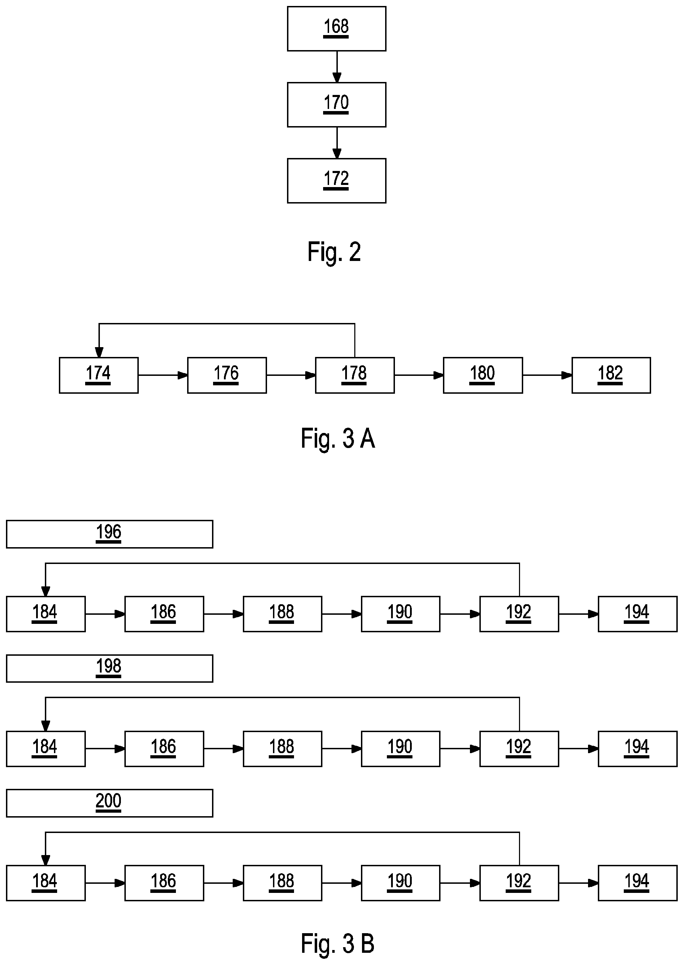

The sensor device 110 further comprises at least one control device 146. The control device 146 is configured for applying a set of operation parameters to the field effect transistor 114 and for monitoring at least one signal value with the field effect transistor 114. The control device 146 is configured for controlling steps ii. and iii. of the method according to any one of the embodiments referring to a method described above or below in further detail, such as according to Figure 2 as will be described below. The control device 146 may comprise at least one source measure unit (SMU) 148. The control device 146 may comprise at least one galvanostat 150. The field effect transistor 114 may be connected to the control device 146. Specifically, the source electrode 116 and the drain electrode 118 may connected directly to the control device 146. The control device 146 may be connected to ground 152. The control device 146 may comprise at least one output 154, specifically at least one analog output 156. The analog output 156 may be connected to at least one contact electrode 158 in contact with the sample 112. The sample 112 may be in contact with the sensing electrode 132. Thus, by using the analog output 156, the control device 146 may be configured for applying a gate potential V_{G} to the gate electrode 120 via the sample 112 and the sensing electrode 132. The control device 146 may comprise at least one processing unit 160 for signal processing and evaluation.

The sensor device 110 may further comprise at least one fluid channel 162. The sensing electrode 132 may be disposed to be in contact with the sample 112 within the fluid channel 162. The sample 112 may flow through the fluid channel 162 as indicated by arrows 164. The sensing electrode 132 may be inserted into the fluid channel 162, such that the sample 112 passes the sensing electrode 132 when flowing through the fluid channel 162. The sensor device 110 may further comprise at least one fluid pump 166 for transporting the sample 112 through the fluid channel 162.

Figure 2 shows a flow chart of an exemplary embodiment of a method of determining the concentration of at least one analyte in a sample 112. The method comprises:
i. (denoted with reference number 168) providing at least one sensor device 110, the sensor device 110 comprising
   - at least one field effect transistor 114 having at least one source electrode 116, at least one drain electrode 118 and at least one gate electrode 120, specifically at least one MOSFET 128,
   - at least one sensing electrode 132 configured for being in contact with the sample 112, the sensing electrode 132 being at least one of electrically connected to the gate electrode 120 of the field effect transistor 114 or integrated into the gate electrode 120 of the field effect transistor 114, and
   - at least one control device 146, the control device 146 being configured for applying operation parameters to the field effect transistor 114 and for monitoring at least one signal value with the field effect transistor 114;
ii. (denoted with reference number 170) at least one parameter selection step comprising selecting a set of operation parameters of the field effect transistor 114 for at least one subsequent measurement step, the parameter selection step comprising performing a plurality of evaluation measurements with the field effect transistor 114 by using various sets of operation parameter candidates and by selecting the set of operation parameters in accordance with at least one optimization criterion monitored during the evaluation measurements; and
iii. (denoted with reference number 172) at least one measurement step comprising detecting the concentration of the analyte by applying the set of operation parameters selected in step ii. to the field effect transistor 114 and by determining at least one signal value with the field effect transistor 114.

The method steps may be performed in the given order. It shall be noted, however, that a different order may also be possible. Further, one or more of the method steps may be performed once or repeatedly. Further, two or more of the method steps may be performed simultaneously or in a timely overlapping fashion. The method may comprise further method steps which are not listed.

The set of operation parameters selected in step ii. may comprise at least two operation parameters selected from the group consisting of: a gate potential V_{G} of the field effect transistor 114; a drain-source-current I_{DS} of the field effect transistor 114. The sensor device 110 may be configured for applying a gate potential V_{G} to the gate electrode 120 of the field effect transistor 114 via the sample 112 and the sensing electrode 132. The sensor device 110 may further be configured for monitoring a drain-source-voltage V_{DS} required for achieving a predefined drain-source-current I_{DS}. The control device 110 may comprise at least one feedback loop for controlling the drain-source-voltage V_{DS} required for achieving the drain-source-current I_{DS}. As said, the control device 110 may specifically comprise at least one galvanostat 150. Thus, the control device 110 may specifically be configured for keeping the drain-source current I_{DS} as predefined, specifically constant. The set of operation parameters selected in step ii. may comprise a gate potential V_{G} of the field effect transistor 114 to be applied to the sample 112 and a drain-source-current I_{DS} of the field effect transistor 114 to be applied to the field effect transistor 114 during detecting the concentration of the analyte. Step iii. may comprise determining a drain-source-voltage V_{DS} required for achieving the drain-source-current I_{DS} as the signal value. Step iii. may further comprise deriving the concentration of the analyte from the drain-source-voltage V_{DS}.

The optimization criterion may be related to at least one measurable optimization criterion value. The optimization criterion value may be selected from the group consisting of: a signal-to-noise-ratio SNR; a signal intensity; a signal noise; a signal drift. The set of operation parameters in step ii. may be selected by selecting operation parameters of an evaluation measurement resulting in one of a maximum optimization criterion value and a minimum optimization criterion value monitored during the evaluation measurements. The set of operation parameters in step ii. may be selected by selecting operation parameters of an evaluation measurement resulting in a maximum signal-to-noise-ratio SNR.

Performing the evaluation measurements in step ii. comprises subsequently performing individual evaluation measurements. Each evaluation measurement may comprise varying one operation parameter while keeping further operation parameters constant. At least one evaluation measurement may comprise varying a gate potential V_{G} of the field effect transistor 114, specifically while keeping a drain-source-current I_{DS} of the field effect transistor 114 constant. At least one evaluation measurement may comprise varying a drain-source-current I_{DS} of the field effect transistor 114, specifically while keeping a gate potential V_{G} of the field effect transistor 114 constant. Step ii. comprises selecting a first operation parameter for step iii. by performing at least one first evaluation measurement comprising varying the first operation parameter while keeping further operation parameters constant. Step ii. further comprises keeping the selected first operation parameter constant in at least one further evaluation measurement performed for selecting at least one further operation parameter for step iii.. Specifically, the first operation parameter may comprise a gate potential V_{G} of the field effect transistor 114 and the further operation parameter may comprise a drain-source-current I_{DS} of the field effect transistor 114.

Step ii. comprises performing the evaluation measurements under experimental conditions corresponding to step iii.. Specifically, step ii. comprises performing the evaluation measurements by using at least one of the same sample 112 as in step ii. The method may at least partially be computer-implemented, specifically at least one of steps ii. and iii. Referring to the computer-implemented aspects of the invention, one or more of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing samples and/or certain aspects of performing actual measurements.

As will be exemplified in further detail below with respect to Figures 3A-3C, step ii. may further comprise performing at least one calibration determining a relation between the concentration of the analyte and the signal value of the field effect transistor 114. Step ii. may further comprise performing at least one calibration determining a relation between the concentration of the analyte and the signal value of the field effect transistor 114. The calibration may comprise determining a sensitivity of the sensor device 110. The calibration may comprise using at least one calibrator, specifically a set comprising a plurality of different calibrators. Step ii. may be re-performed when changing a measurement range in step iii.. For each individual measurement range, at least one individual calibration may be performed. In other words, the method may comprise performing at least one calibration determining a relation between the concentration of the analyte and the signal value of the field effect transistor 114 for each measurement range.

Figures 3A-3C show flow charts of exemplary embodiments of a calibration of a sensor device 110 for determining the concentration of at least one analyte in a sample 112. Figure 3A illustrates an exemplary factory calibration of the sensor device 110. The factory calibration may comprise the following factory calibration steps:
a) (denoted with reference number 174) selecting a gate potential V_{G};
b) (denoted with reference number 176) selecting a drain-source-current I_{DS};
c) (denoted with reference number 178) determining a sensitivity and a signal-to-noise-ratio SNR;
d) (denoted with reference number 180) characterizing the field effect transistor 114; and
e) (denoted with reference number 182) saving factory field effect transistor settings.

The factory calibration steps may be performed in the given order. It shall be noted, however, that a different order may also be possible. Further, one or more of the factory calibration steps may be performed once or repeatedly. Further, two or more of the factory calibration steps may be performed simultaneously or in a timely overlapping fashion. The factory calibration may comprise further steps which are not listed.

Specifically, factory calibration steps a) to c) may be performed iteratively. Thus, after determining the sensitivity and the signal-to-noise-ratio SNR based on the selected gate potential V_{G} and the selected drain-source-current I_{DS}, a new gate potential V_{G} and a new drain-source-current I_{DS} may be selected, e.g. by using at least one optimization algorithm. The optimization algorithm may specifically comprise selecting different values for the gate potential V_{G} and/or the drain-source-current I_{DS}, e.g. in an ascending order in a predefined interval such as in a measurement range of the sensor device 110. As an example, different values for the gate potential V_{G} and/or the drain-source-current I_{DS} may be selected by using at least one predefined step size for successively increasing or decreasing the values for the gate potential V_{G} and/or the drain-source-current I_{DS}. Additionally or alternatively, predefined distinct values for the gate potential V_{G} and/or the drain-source-current I_{DS} may for example be selected successively. The optimization algorithm may specifically comprise comparing the sensitivities and/or the signal-to-noise-ratios SNR for the different values for the gate potential V_{G} and/or the drain-source-current I_{DS} among each other. Thus, the optimization algorithm may specifically comprise finding an optimized gate potential V_{G} and/or an optimized drain-source-current I_{DS}, specifically a gate potential V_{G} and/or a drain-source-current I_{DS} yielding an optimized sensitivity and/or an optimized signal-to-noise-ratio SNR, e.g. a maximal sensitivity and/or a maximal signal-to-noise-ratio SNR. Generally, other options for the optimization algorithm may also be feasible. Once the optimized gate potential V_{G} and the optimized drain-source-current I_{DS} are found in factory calibration steps a) to c), the field effect transistor 114, which may specifically be a MOSFET 128, may be characterized, e.g. be determining at least one of a field effect mobility, an on-off-ratio and a threshold voltage. Finally, an outcome of the characterization of the field effect transistor 114 may be saved as factory field effect transistor settings.

Figure 3B illustrates an exemplary initial calibration of the sensor device 110 for three measurement ranges. Generally, less or also more measurement ranges may be feasible. The initial calibration may comprise the following initial calibration steps:
a) (denoted with reference number 184) selecting a gate potential V_{G};
b) (denoted with reference number 186) selecting a drain-source-current I_{DS};
c) (denoted with reference number 188) using a first calibrator;
d) (denoted with reference number 190) using a second calibrator;
e) (denoted with reference number 192) determining a sensitivity and a signal-to-noise-ratio; and
f) (denoted with reference number 194) saving an optimized sensitivity and an optimized signal-to-noise-ratio SNR.

The initial calibration steps may be performed in the given order. It shall be noted, however, that a different order may also be possible. Further, one or more of the initial calibration steps may be performed once or repeatedly. Further, two or more of the initial calibration steps may be performed simultaneously or in a timely overlapping fashion. The initial calibration may comprise further steps which are not listed.

Specifically, initial calibration steps a) to e) may be performed iteratively. Thus, after determining the sensitivity and the signal-to-noise-ratio SNR based on the selected gate potential V_{G} and the selected drain-source-current I_{DS} by using the first calibrator and the second calibrator, a new gate potential V_{G} and a new drain-source-current I_{DS} may be selected, e.g. by using at least one optimization algorithm. As already indicated, the optimization algorithm may specifically comprise selecting different values for the gate potential V_{G} and/or the drain-source-current I_{DS}, e.g. in an ascending order in a predefined interval such as in a measurement range of the sensor device 110. As an example, different values for the gate potential V_{G} and/or the drain-source-current I_{DS} may be selected by using at least one predefined step size for successively increasing or decreasing the values for the gate potential V_{G} and/or the drain-source-current I_{DS}. Additionally or alternatively, predefined distinct values for the gate potential V_{G} and/or the drain-source-current I_{DS} may for example be selected successively. The different values for the gate potential V_{G} and/or the drain-source-current I_{DS} may specifically be used at least on the first calibrator and/or the second calibrator. The optimization algorithm may specifically comprise comparing the sensitivities and/or the signal-to-noise-ratios SNR for the different values for the gate potential V_{G} and/or the drain-source-current I_{DS} among each other. Thus, the optimization algorithm may specifically comprise finding an optimized gate potential V_{G} and/or an optimized drain-source-current I_{DS}, specifically a gate potential V_{G} and/or a drain-source-current I_{DS} yielding an optimized sensitivity and/or an optimized signal-to-noise-ratio SNR, e.g. a maximal sensitivity and/or a maximal signal-to-noise-ratio SNR. Generally, other options for the optimization algorithm may also be feasible. Once the optimized gate potential V_{G} and the optimized drain-source-current I_{DS} are found in initial calibration steps a) to e), the optimized sensitivity and the optimized signal-to-noise-ratio SNR may be saved.

The initial calibration may be performed individually for each measurement range. A first measurement range is denoted with reference number 196, a second measurement range is denoted with reference number 198 and a third measurement range is denoted with reference number 200. The initial calibration may be performed initially, when sensor device 110 is installed and put into operation on site, e.g. in a laboratory. Afterwards, further calibrations may be performed in regular or irregular time intervals, e.g. daily.

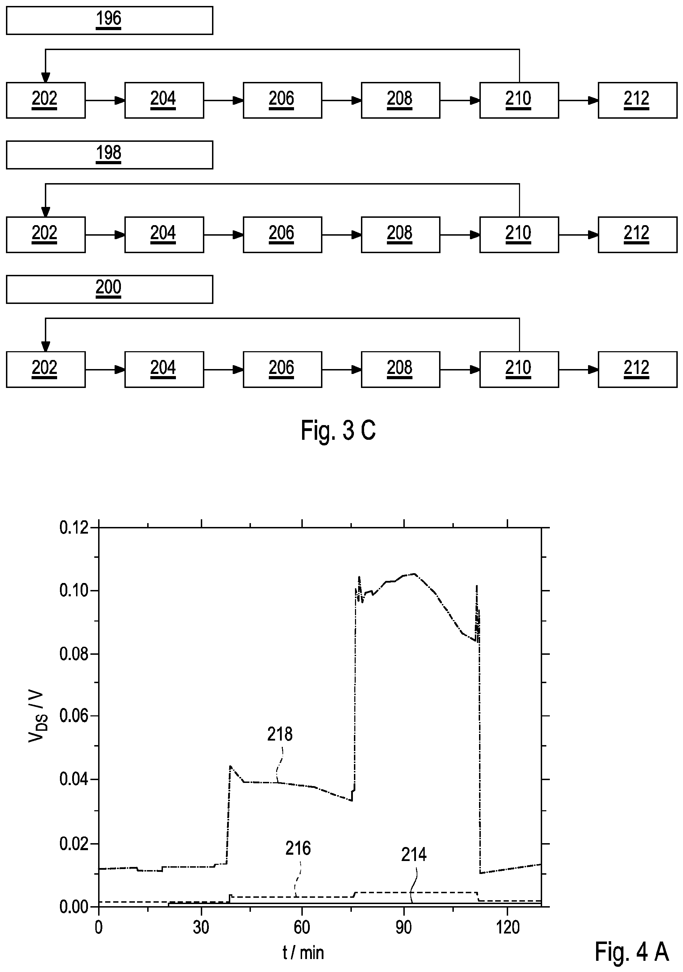

Figure 3C illustrates an exemplary daily calibration of the sensor device 110 for the three measurement ranges. As an example, the daily calibration may be performed analogous to the initial calibration. However, changes or adaptions may in principle also be feasible. Thus, the daily calibration may comprise the following daily calibration steps:
a) (denoted with reference number 202) selecting a gate potential V_{G};
b) (denoted with reference number 204) selecting a drain-source-current I_{DS};
c) (denoted with reference number 206) using a first calibrator;
d) (denoted with reference number 208) using a second calibrator;
e) (denoted with reference number 210) determining a sensitivity and a signal-to-noise-ratio; and
f) (denoted with reference number 212) saving an optimized sensitivity and an optimized signal-to-noise-ratio SNR.

The daily calibration steps may be performed in the given order. It shall be noted, however, that a different order may also be possible. Further, one or more of the daily calibration steps may be performed once or repeatedly. Further, two or more of the daily calibration steps may be performed simultaneously or in a timely overlapping fashion. The daily calibration may comprise further steps which are not listed.

Specifically, daily calibration steps a) to e) may be performed iteratively. Thus, after determining the sensitivity and the signal-to-noise-ratio SNR based on the selected gate potential V_{G} and the selected drain-source-current I_{DS} by using the first calibrator and the second calibrator, a new gate potential V_{G} and a new drain-source-current I_{DS} may be selected, e.g. by using at least one optimization algorithm. As already indicated, the optimization algorithm may specifically comprise selecting different values for the gate potential V_{G} and/or the drain-source-current I_{DS}, e.g. in an ascending order in a predefined interval such as in a measurement range of the sensor device 110. As an example, different values for the gate potential V_{G} and/or the drain-source-current I_{DS} may be selected by using at least one predefined step size for successively increasing or decreasing the values for the gate potential V_{G} and/or the drain-source-current I_{DS}. Additionally or alternatively, predefined distinct values for the gate potential V_{G} and/or the drain-source-current I_{DS} may for example be selected successively. The different values for the gate potential V_{G} and/or the drain-source-current I_{DS} may specifically be used at least on the first calibrator and/or the second calibrator. The optimization algorithm may specifically comprise comparing the sensitivities and/or the signal-to-noise-ratios SNR for the different values for the gate potential V_{G} and/or the drain-source-current I_{DS} among each other. Thus, the optimization algorithm may specifically comprise finding an optimized gate potential V_{G} and/or an optimized drain-source-current I_{DS}, specifically a gate potential V_{G} and/or a drain-source-current I_{DS} yielding an optimized sensitivity and/or an optimized signal-to-noise-ratio SNR, e.g. a maximal sensitivity and/or a maximal signal-to-noise-ratio SNR. Generally, other options for the optimization algorithm may also be feasible. Once the optimized gate potential V_{G} and the optimized drain-source-current I_{DS} are found in daily calibration steps a) to e), the optimized sensitivity and the optimized signal-to-noise-ratio SNR may be saved. As before, the daily calibration may be performed individually for each measurement range. As can be seen, at least one of the factory calibration, the initial calibration and the daily calibration may comprise selecting a set of operation parameters, in particular specific gate potentials V_{G} and drain-source-currents I_{DS}.

Figures 4A-4E show experimental results of evaluation measurements of an exemplary embodiment of step ii. of a method of determining the concentration of at least one analyte in a sample 112 plus a corresponding concentration detection of potassium. Thus, Figures 4A-4D specifically show results of exemplary evaluation measurements for selecting a set of operation parameters. The evaluation measurements were performed by using an EGFET 130 comprising a MOSFET 128 by Microchip Technolgy Inc. and an extended gate electrode 134 as a sensing electrode 132. An Ag/AgCl contact electrode 158 was used for applying constant gate potentials V_{g}. Electrical measurements were carried out with a multichannel control device 146 of the type IVIUM n-stat comprising a galvanostat 150. The drain-source-voltage V_{DS} needed to keep a constant drain-source-current I_{DS} was monitored over time. Samples 112 were passed through the sensor electrodes using a fluid pump 166 of the type Aladdin AL4000-220Z by World Precision Instruments, Germany.

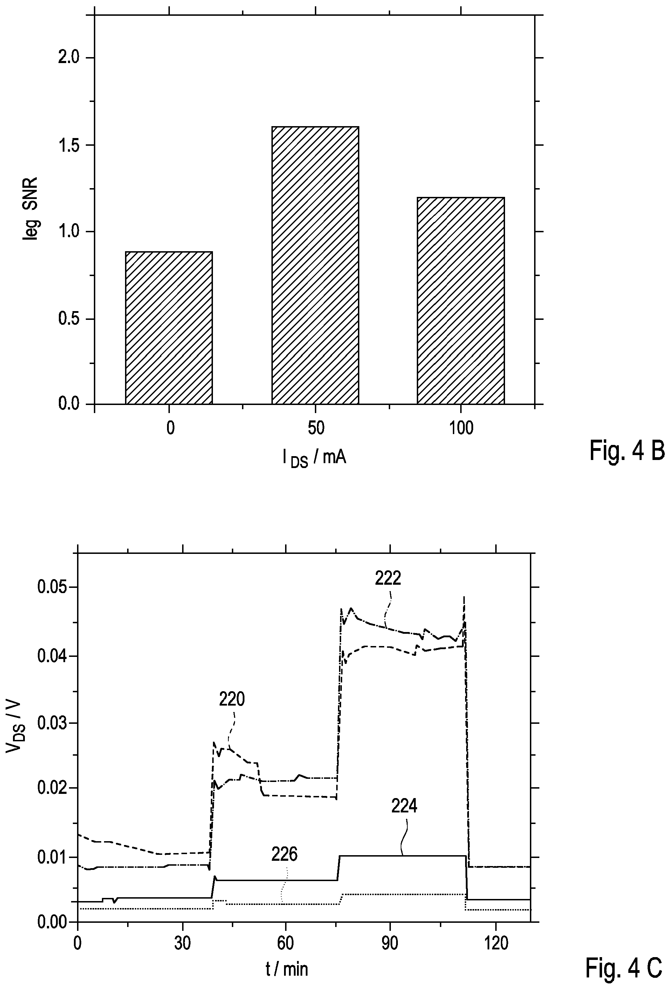

For the experimental results shown in Figures 4A-4B, the gate potential V_{G} was fixed at - 600 mV and the drain-source-current I_{DS} was altered from 5 nA over 50 nA to 100 nA. Figure 4A shows the drain-source-voltages V_{DS} required for achieving the respective drain-source-current I_{DS} at the fixed gate potential V_{G} within a time period over more than 120 minutes, wherein the concentration of the analyte in the sample 112 was altered after approximately 35 min, 75 min and 115 min. The drain-source-voltage V_{DS} required for achieving a drain-source-current I_{DS} of 5 nA at the fixed gate potential V_{G} of -600 mV is denoted with reference sign 214. The drain-source-voltage V_{DS} required for achieving a drain-source-current I_{DS} of 50 nA at the fixed gate potential V_{G} of -600 mV is denoted with reference sign 216. The drain-source-voltage V_{DS} required for achieving a drain-source-current I_{DS} of 100 nA at the fixed gate potential V_{G} of -600 mV is denoted with reference sign 218. Distinct levels are recognizable in Figure 4A each time the concentration of the analyte in the sample 112 is altered. However, the respective levels in Figure 4A also show deviations corresponding to a signal-to-noise-ratio SNR. Figure 4B shows the logarithm of the signal-to-noise-ration SNR over the respective drain-source-current I_{DS}, which indicates that the highest signal-to-noise-ration SNR can be found for a drain-source-current I_{DS} of 50 nA.

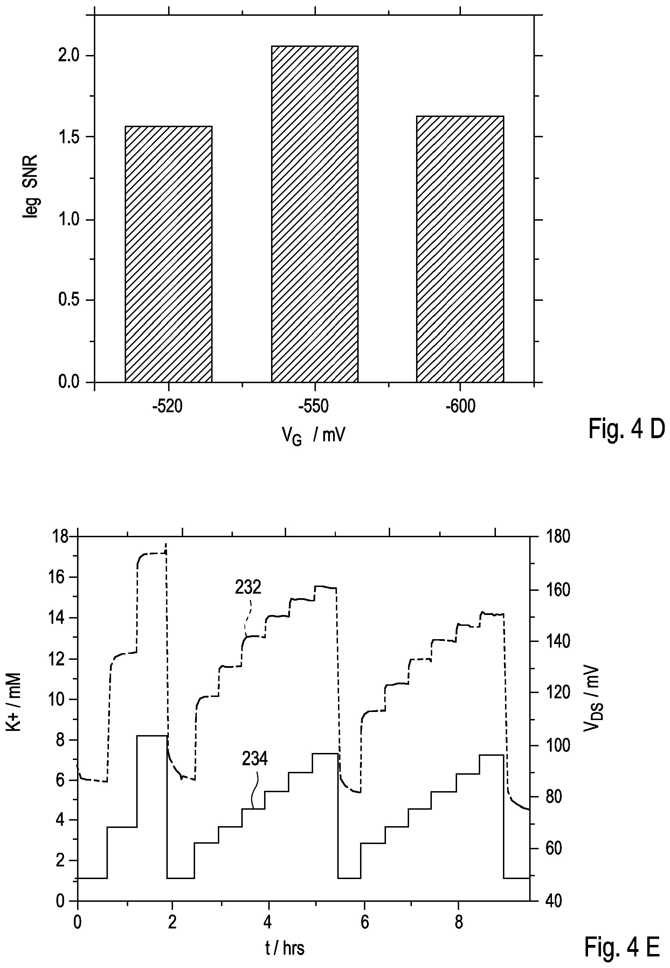

For the experimental results shown in Figures 4C-4D, the drain-source-current I_{DS} was fixed at 50 nA and the gate potential V_{G} was altered. Analogous to before, Figure 4C shows the drain-source-voltages V_{DS} required for achieving the fixed drain-source-current I_{DS} at the respective gate potential V_{G}. The drain-source-voltage V_{DS} required for achieving the fixed drain-source-current I_{DS} of 50 nA at a gate potential V_{G} of -501 mV is denoted with reference sign 220. The drain-source-voltage V_{DS} required for achieving the fixed drain-source-current I_{DS} of 50 nA at a gate potential V_{G} of -520 mV is denoted with reference sign 222. The drain-source-voltage V_{DS} required for achieving the fixed drain-source-current I_{DS} of 50 nA at a gate potential V_{G} of -550 mV is denoted with reference sign 224. The drain-source-voltage V_{DS} required for achieving the fixed drain-source-current I_{DS} of 50 nA at a gate potential V_{G} of -600 mV is denoted with reference sign 226. Again, distinct levels for each concentration of the analyte are recognizable. Potassium was used as analyte. The experiment started with a concentration of 1 mM, which was first increased to 4 mM, then to 8 mM, before it was eventually set back to 1 mM. Figure 4D indicates that the highest signal-to-noise-ratio SNR can be found for a gate potential V_{G} of -550 mV. Thus, with the signal-to-noise-ratio SNR as optimization criterion, the exemplary evaluation measurements overall led to a selection of a drain-source-current I_{DS} of 50 nA and a gate potential V_{G} of -550 mV as operation parameters.

Figure 4E shows exemplary concentration measurements of potassium (K+) over more than 9 hours with the operation parameters as selected above in the evaluation measurements. Thus, gate potential V_{G} and source-drain-current I_{DS} were selected as operation parameters and the source-drain-voltage V_{DS} was monitored which was required for achieving the selected operation parameters, specifically the source-drain-current I_{DS} affected by the potassium concentration. A drain-source-current I_{DS} of 50 nA and a gate potential V_{G} of -550 mV were used. In Figure 4E, the source-drain-voltage V_{DS} is denoted with reference sign 232. The corresponding potassium concentration is denoted with reference sign 234.

### List of reference numbers

- 110: sensor device
- 112: sample
- 114: field effect transistor
- 116: source electrode
- 118: drain electrode
- 120: gate electrode
- 122: semiconductor
- 124: dielectric
- 126: channel
- 128: metal-oxide-semiconductor field effect transistor (MOSFET)
- 130: extended gate field effect transistor (EGFET)
- 132: sensing electrode
- 134: extended gate electrode
- 136: wire
- 138: trace
- 140: functional component
- 142: receptor compound
- 144: ionophore
- 146: control device
- 148: source measure unit
- 150: galvanostat
- 152: ground
- 154: output
- 156: analog output
- 158: contact electrode
- 160: processing unit
- 162: fluid channel
- 164: arrow indicating fluid flow
- 166: fluid pump
- 168: method step i.
- 170: method step ii.
- 172: method step iii.
- 174: factory calibration step a)
- 176: factory calibration step b)
- 178: factory calibration step c)
- 180: factory calibration step d)
- 182: factory calibration step e)
- 184: initial calibration step a)
- 186: initial calibration step b)
- 188: initial calibration step c)
- 190: initial calibration step d)
- 192: initial calibration step e)
- 194: initial calibration step f)
- 196: first measurement range
- 198: second measurement range
- 200: third measurement range
- 202: daily calibration step a)
- 204: daily calibration step b)
- 206: daily calibration step c)
- 208: daily calibration step d)
- 210: daily calibration step e)
- 212: daily calibration step f)
- 214: V_{DS} required for achieving a I_{DS} of 5 nA at a V_{G} of -600 mV
- 216: V_{DS} required for achieving a I_{DS} of 50 nA at a V_{G} of -600 mV
- 218: V_{DS} required for achieving a I_{DS} of 100 nA at a V_{G} of -600 mV
- 220: V_{DS} required for achieving a I_{DS} of 50 nA at a V_{G} of -501 mV
- 222: V_{DS} required for achieving a I_{DS} of 50 nA at a V_{G} of -520 mV
- 224: V_{DS} required for achieving a I_{DS} of 50 nA at a V_{G} of -550 mV
- 226: V_{DS} required for achieving a I_{DS} of 50 nA at a V_{G} of -600 mV
- 232: V_{DS} for first exemplary embodiment of parts of sensor device
- 234: potassium concentration for first exemplary embodiment of parts of sensor device

## Claims

1. A method of determining the concentration of at least one analyte in a sample (112) the method comprising:
i. providing at least one sensor device (110), the sensor device (110) comprising
- at least one field effect transistor (114) having at least one source electrode (116), at least one drain electrode (118) and at least one gate electrode (120),
- at least one sensing electrode (132) configured for being in contact with the sample (112), the sensing electrode (132) being at least one of electrically connected to the gate electrode (120) of the field effect transistor (114) or integrated into the gate electrode (120) of the field effect transistor (114), and
- at least one control device (146), the control device (146) being configured for applying operation parameters to the field effect transistor (114) and for monitoring at least one signal value with the field effect transistor (114);
ii. at least one parameter selection step comprising selecting a set of operation parameters of the field effect transistor (114) for at least one subsequent measurement step, the parameter selection step comprising performing a plurality of evaluation measurements with the field effect transistor (114) by using various sets of operation parameter candidates and by selecting the set of operation parameters in accordance with at least one optimization criterion monitored during the evaluation measurements; and
iii. at least one measurement step comprising detecting the concentration of the analyte by applying the set of operation parameters selected in step ii. to the field effect transistor (114) and by determining at least one signal value with the field effect transistor (114),
wherein step ii. comprises selecting a first operation parameter for step iii. by performing at least one first evaluation measurement comprising varying the first operation parameter while keeping further operation parameters constant, wherein step ii. further comprises keeping the selected first operation parameter constant in at least one further evaluation measurement performed for selecting at least one further operation parameter for step iii., wherein step ii. comprises performing the evaluation measurements under experimental conditions corresponding to step iii.,
**characterised in that** step ii. comprises performing the evaluation measurements by using the same sample as in step iii..

2. The method according to the preceding claim, wherein the first operation parameter comprises a gate potential V_{G} of the field effect transistor and the further operation parameter comprises a drain-source-current I_{DS} of the field effect transistor.

3. The method according to any one of the preceding claims, wherein the set of operation parameters selected in step ii. comprises at least two operation parameters selected from the group consisting of: a gate potential V_{G} of the field effect transistor (114); a drain-source-current I_{DS} of the field effect transistor (114).

4. The method according to any one of the preceding claims, wherein the sensor device (110) is configured for applying a gate potential V_{G} to the gate electrode (120) of the field effect transistor (114) via the sample (112) and the sensing electrode (132) and further for monitoring a drain-source-voltage V_{DS} required for achieving a predefined drain-source-current I_{DS}, wherein the control device (110) comprises at least one feedback loop for controlling the drain-source-voltage V_{DS} required for achieving the drain-source-current I_{DS}.

5. The method according to any one of the preceding claims, wherein the set of operation parameters selected in step ii. comprises a gate potential V_{G} of the field effect transistor (114) to be applied to the sample (112) and a drain-source-current I_{DS} of the field effect transistor (114) to be applied to the field effect transistor (114) during detecting the concentration of the analyte, wherein step iii. comprises determining a drain-source-voltage V_{DS} required for achieving the drain-source-current I_{DS} as the signal value and further comprises deriving the concentration of the analyte from the drain-source-voltage V_{DS}.

6. The method according to any one of the preceding claims, wherein the optimization criterion is related to at least one measurable optimization criterion value, wherein the optimization criterion value is selected from the group consisting of: a signal-to-noise-ratio SNR; a signal intensity; a signal noise; a signal drift.

7. The method according to any one of the preceding claims, wherein performing the evaluation measurements in step ii. comprises subsequently performing individual evaluation measurements, wherein each evaluation measurement comprises varying one operation parameter while keeping further operation parameters constant.

8. The method according to the preceding claim, wherein at least one evaluation measurement comprises varying a gate potential V_{G} of the field effect transistor, wherein at least one evaluation measurement comprises varying a drain-source-current I_{DS} of the field effect transistor.

9. The method according to any one of the preceding claims, wherein step ii. is re-performed when changing a measurement range in step iii..

10. The method according to any one of the preceding claims, wherein step ii. further comprises performing at least one calibration determining a relation between the concentration of the analyte and the signal value of the field effect transistor (114), wherein the calibration comprises determining a sensitivity of the sensor device (110).

11. The method according to any one of the preceding claims, wherein the field effect transistor (114) is an extended gate field effect transistor (EGFET) (130), wherein the sensing electrode (132) is an extended gate electrode (134).

12. The method according to any one of the preceding claims, wherein the sensing electrode (134) comprises at least one functional component (140) on its surface, wherein the functional component (140) is configured for interacting with the analyte, wherein the functional component (140) comprises at least one of:
- at least one receptor compound (142), the receptor compound (142) being capable of binding the at least one analyte; and
- at least one ionophore (144).

13. The method according to any one of the preceding claims, wherein the sample (112) comprises a bodily fluid.

14. The method according to any one of the preceding claims, wherein the method is at least partially computer-implemented.

## Patentansprüche

1. Verfahren zum Bestimmen der Konzentration von mindestens einem Analyten in einer Probe (112), wobei das Verfahren Folgendes umfasst:
i. Bereitstellen von mindestens einer Sensorvorrichtung (110), wobei die Sensorvorrichtung (110) Folgendes umfasst:
- mindestens einen Feldeffekttransistor (114) mit mindestens einer Source-Elektrode (116), mindestens einer Drain-Elektrode (118) und mindestens einer Gate-Elektrode (120),
- mindestens eine Messelektrode (132), die so konfiguriert ist, dass sie mit der Probe (112) in Kontakt steht, wobei die Messelektrode (132) elektrisch mit der Gate-Elektrode (120) des Feldeffekttransistors (114) verbunden und/oder in die Gate-Elektrode (120) des Feldeffekttransistors (114) integriert ist, und
- mindestens eine Steuervorrichtung (146), wobei die Steuervorrichtung (146) so konfiguriert ist, dass sie Betriebsparameter an den Feldeffekttransistor (114) anlegt und mindestens einen Signalwert mit dem Feldeffekttransistor (114) überwacht;
ii. mindestens einen Parameterauswahlschritt, der das Auswählen eines Satzes von Betriebsparametern des Feldeffekttransistors (114) für mindestens einen nachfolgenden Messschritt umfasst, wobei der Parameterauswahlschritt das Durchführen einer Vielzahl von Evaluierungsmessungen mit dem Feldeffekttransistor (114) unter Verwendung verschiedener Sätze von Betriebsparameterkandidaten und durch Auswählen des Satzes von Betriebsparametern gemäß mindestens einem während der Evaluierungsmessungen überwachten Optimierungskriterium umfasst; und
iii. mindestens einen Messschritt, der das Erfassen der Konzentration des Analyten durch Anlegen des in Schritt ii. ausgewählten Satzes von Betriebsparametern an den Feldeffekttransistor (114) und durch Bestimmen mindestens eines Signalwerts mit dem Feldeffekttransistor (114) umfasst,
wobei Schritt ii. das Auswählen eines ersten Betriebsparameters für Schritt iii. durch Durchführen mindestens einer ersten Evaluierungsmessung umfasst, die das Variieren des ersten Betriebsparameters umfasst, während weitere Betriebsparameter konstant gehalten werden, wobei Schritt ii. ferner das Konstanthalten des ausgewählten ersten Betriebsparameters in mindestens einer weiteren Evaluierungsmessung umfasst, die zum Auswählen mindestens eines weiteren Betriebsparameters für Schritt iii. durchgeführt wird, wobei Schritt ii. das Durchführen der Evaluierungsmessungen unter experimentellen Bedingungen umfasst, die Schritt iii. entsprechen, **dadurch gekennzeichnet, dass** Schritt ii. das Durchführen der Evaluierungsmessungen unter Verwendung derselben Probe wie in Schritt iii. umfasst.

2. Verfahren nach dem vorhergehenden Anspruch, wobei der erste Betriebsparameter ein Gate-Potential V_{G} des Feldeffekttransistors umfasst und der weitere Betriebsparameter einen Drain-Source-Strom I_{DS} des Feldeffekttransistors umfasst.

3. Verfahren nach einem der vorhergehenden Ansprüche, wobei der in Schritt ii. ausgewählte Satz von Betriebsparametern mindestens zwei Betriebsparameter umfasst, die ausgewählt sind aus der Gruppe bestehend aus: einem Gate-Potential V_{G} des Feldeffekttransistors (114); einem Drain-Source-Strom I_{DS} des Feldeffekttransistors (114).

4. Verfahren nach einem der vorhergehenden Ansprüche, wobei die Sensorvorrichtung (110) so konfiguriert ist, dass sie ein Gate-Potential V_{G} an die Gate-Elektrode (120) des Feldeffekttransistors (114) über die Probe (112) und die Messelektrode (132) anlegt und ferner eine Drain-Source-Spannung V_{DS} überwacht, die erforderlich ist, um einen vordefinierten Drain-Source-Strom I_{DS} zu erreichen, wobei die Steuervorrichtung (110) mindestens eine Rückkopplungsschleife zum Steuern der Drain-Source-Spannung V_{DS} umfasst, die erforderlich ist, um den Drain-Source-Strom I_{DS} zu erreichen.

5. Verfahren nach einem der vorhergehenden Ansprüche, wobei der in Schritt ii. ausgewählte Satz von Betriebsparametern ein Gate-Potential V_{G} des Feldeffekttransistors (114), das an die Probe (112) angelegt werden soll, und einen Drain-Source-Strom I_{DS} des Feldeffekttransistors (114) umfasst, der an den Feldeffekttransistor (114) angelegt werden soll, während die Konzentration des Analyten erfasst wird, wobei Schritt iii. das Bestimmen einer Drain-Source-Spannung V_{DS} umfasst, die erforderlich ist, um den Drain-Source-Strom I_{DS} als den Signalwert zu erreichen, und ferner das Ableiten der Konzentration des Analyten aus der Drain-Source-Spannung V_{DS} umfasst.

6. Verfahren nach einem der vorhergehenden Ansprüche, wobei das Optimierungskriterium mit mindestens einem messbaren Optimierungskriteriumwert in Beziehung steht, wobei der Optimierungskriteriumwert ausgewählt ist aus der Gruppe bestehend aus: einem Signal-Rausch-Verhältnis SNR; einer Signalintensität; einem Signalrauschen; einer Signaldrift.

7. Verfahren nach einem der vorhergehenden Ansprüche, wobei das Durchführen der Evaluierungsmessungen in Schritt ii. das anschließende Durchführen einzelner Evaluierungsmessungen umfasst, wobei jede Evaluierungsmessung das Variieren eines Betriebsparameters umfasst, während weitere Betriebsparameter konstant gehalten werden.

8. Verfahren nach dem vorhergehenden Anspruch, wobei mindestens eine Evaluierungsmessung das Variieren eines Gate-Potentials V_{G} des Feldeffekttransistors umfasst, wobei mindestens eine Evaluierungsmessung das Variieren eines Drain-Source-Stroms I_{DS} des Feldeffekttransistors umfasst.

9. Verfahren nach einem der vorhergehenden Ansprüche, wobei Schritt ii. erneut durchgeführt wird, wenn ein Messbereich in Schritt iii. geändert wird.

10. Verfahren nach einem der vorhergehenden Ansprüche, wobei Schritt ii. ferner das Durchführen mindestens einer Kalibrierung umfasst, die eine Beziehung zwischen der Konzentration des Analyten und dem Signalwert des Feldeffekttransistors (114) bestimmt, wobei die Kalibrierung das Bestimmen einer Empfindlichkeit der Sensorvorrichtung (110) umfasst.

11. Verfahren nach einem der vorhergehenden Ansprüche, wobei der Feldeffekttransistor (114) ein Feldeffekttransistor mit erweitertem Gate (EGFET) (130) ist, wobei die Messelektrode (132) eine erweiterte Gate-Elektrode (134) ist.

12. Verfahren nach einem der vorhergehenden Ansprüche, wobei die Messelektrode (134) mindestens eine funktionelle Komponente (140) auf ihrer Oberfläche umfasst, wobei die funktionelle Komponente (140) zum Interagieren mit dem Analyten konfiguriert ist, wobei die funktionelle Komponente (140) mindestens eines von Folgendem umfasst:
- mindestens einer Rezeptorverbindung (142), wobei die Rezeptorverbindung (142) in der Lage ist, den mindestens einen Analyten zu binden; und
- mindestens einem Ionophor (144).

13. Verfahren nach einem der vorhergehenden Ansprüche, wobei die Probe (112) eine Körperflüssigkeit umfasst.

14. Verfahren nach einem der vorhergehenden Ansprüche, wobei das Verfahren mindestens teilweise computerimplementiert ist.

## Revendications

1. Procédé pour déterminer la concentration d'au moins un analyte dans un échantillon (112), le procédé comprenant :
i. la fourniture d'au moins un dispositif de détection (110), le dispositif de détection (110) comprenant
- au moins un transistor à effet de champ (114) ayant au moins une électrode de source (116), au moins une électrode de drain (118) et au moins une électrode de grille (120),
- au moins une électrode de détection (132) configurée pour être en contact avec l'échantillon (112), l'électrode de détection (132) étant connectée électriquement à l'électrode de grille (120) du transistor à effet de champ (114) et/ou intégrée dans l'électrode de grille (120) du transistor à effet de champ (114), et
- au moins un dispositif de commande (146), le dispositif de commande (146) étant configuré pour appliquer des paramètres de fonctionnement au transistor à effet de champ (114) et pour surveiller au moins une valeur de signal avec le transistor à effet de champ (114) ;
ii. au moins une étape de sélection de paramètres comprenant la sélection d'un ensemble de paramètres de fonctionnement du transistor à effet de champ (114) pour au moins une étape de mesure ultérieure, l'étape de sélection de paramètres comprenant la réalisation d'une pluralité de mesures d'évaluation avec le transistor à effet de champ (114) en utilisant divers ensembles de candidats de paramètres de fonctionnement et en sélectionnant l'ensemble de paramètres de fonctionnement conformément à au moins un critère d'optimisation surveillé pendant les mesures d'évaluation ; et
iii. au moins une étape de mesure comprenant la détection de la concentration de l'analyte en appliquant l'ensemble de paramètres de fonctionnement sélectionné dans l'étape ii. au transistor à effet de champ (114) et en déterminant au moins une valeur de signal avec le transistor à effet de champ (114),
dans lequel l'étape ii. comprend la sélection d'un premier paramètre de fonctionnement pour l'étape iii. en réalisant au moins une première mesure d'évaluation comprenant la variation du premier paramètre de fonctionnement tout en maintenant les autres paramètres de fonctionnement constants, dans lequel l'étape ii. comprend en outre le maintien du premier paramètre de fonctionnement sélectionné constant dans au moins une mesure d'évaluation supplémentaire réalisée pour sélectionner au moins un paramètre de fonctionnement supplémentaire pour l'étape iii., dans lequel l'étape ii. comprend la réalisation des mesures d'évaluation dans des conditions expérimentales correspondant à l'étape iii., **caractérisé en ce que** l'étape ii. comprend la réalisation des mesures d'évaluation en utilisant le même échantillon que dans l'étape iii..

2. Procédé selon la revendication précédente, dans lequel le premier paramètre de fonctionnement comprend un potentiel de grille V_{G} du transistor à effet de champ et le paramètre de fonctionnement supplémentaire comprend un courant drain-source I_{DS} du transistor à effet de champ.

3. Procédé selon l'une quelconque des revendications précédentes, dans lequel l'ensemble de paramètres de fonctionnement sélectionnés dans l'étape ii. comprend au moins deux paramètres de fonctionnement choisis dans le groupe constitué par : un potentiel de grille V_{G} du transistor à effet de champ (114) ; un courant drain-source I_{DS} du transistor à effet de champ (114).

4. Procédé selon l'une quelconque des revendications précédentes, dans lequel le dispositif de détection (110) est configuré pour appliquer un potentiel de grille V_{G} à l'électrode de grille (120) du transistor à effet de champ (114) par l'intermédiaire de l'échantillon (112) et de l'électrode de détection (132) et en outre pour surveiller une tension drain-source V_{DS} nécessaire pour obtenir un courant drain-source I_{DS} prédéfini, dans lequel le dispositif de commande (110) comprend au moins une boucle de rétroaction pour commander la tension drain-source V_{DS} nécessaire pour obtenir le courant drain-source I_{DS}.

5. Procédé selon l'une quelconque des revendications précédentes, dans lequel l'ensemble de paramètres de fonctionnement sélectionné dans l'étape ii. comprend un potentiel de grille V_{G} du transistor à effet de champ (114) à appliquer à l'échantillon (112) et un courant drain-source I_{DS} du transistor à effet de champ (114) à appliquer au transistor à effet de champ (114) pendant la détection de la concentration de l'analyte, dans lequel l'étape iii. comprend la détermination d'une tension drain-source V_{DS} nécessaire pour obtenir le courant drain-source I_{DS} en tant que valeur de signal et comprend en outre la dérivation de la concentration de l'analyte à partir de la tension drain-source V_{DS}.

6. Procédé selon l'une quelconque des revendications précédentes, dans lequel le critère d'optimisation est lié à au moins une valeur de critère d'optimisation mesurable, dans lequel la valeur de critère d'optimisation est choisie dans le groupe constitué par : un rapport signal sur bruit SNR ; une intensité de signal ; un bruit de signal ; une dérive de signal.

7. Procédé selon l'une quelconque des revendications précédentes, dans lequel la réalisation des mesures d'évaluation dans l'étape ii. comprend la réalisation ultérieure de mesures d'évaluation individuelles, dans lequel chaque mesure d'évaluation comprend la variation d'un paramètre de fonctionnement tout en maintenant les autres paramètres de fonctionnement constants.

8. Procédé selon la revendication précédente, dans lequel au moins une mesure d'évaluation comprend la variation d'un potentiel de grille V_{G} du transistor à effet de champ, dans lequel au moins une mesure d'évaluation comprend la variation d'un courant drain-source I_{DS} du transistor à effet de champ.

9. Procédé selon l'une quelconque des revendications précédentes, dans lequel l'étape ii. est réalisée à nouveau lors du changement d'une plage de mesure dans l'étape iii..

10. Procédé selon l'une quelconque des revendications précédentes, dans lequel l'étape ii. comprend en outre la réalisation d'au moins un étalonnage déterminant une relation entre la concentration de l'analyte et la valeur de signal du transistor à effet de champ (114), dans lequel l'étalonnage comprend la détermination d'une sensibilité du dispositif de détection (110).

11. Procédé selon l'une quelconque des revendications précédentes, dans lequel le transistor à effet de champ (114) est un transistor à effet de champ à grille étendue (EGFET) (130), dans lequel l'électrode de détection (132) est une électrode de grille étendue (134).

12. Procédé selon l'une quelconque des revendications précédentes, dans lequel l'électrode de détection (134) comprend au moins un composant fonctionnel (140) sur sa surface, dans lequel le composant fonctionnel (140) est configuré pour interagir avec l'analyte, dans lequel le composant fonctionnel (140) comprend au moins un parmi :
- au moins un composé récepteur (142), le composé récepteur (142) étant capable de se lier à l'au moins un analyte ; et
- au moins un ionophore (144).

13. Procédé selon l'une quelconque des revendications précédentes, dans lequel l'échantillon (112) comprend un fluide corporel.

14. Procédé selon l'une quelconque des revendications précédentes, dans lequel le procédé est au moins partiellement mis en œuvre par ordinateur.
